(19) Europäisches Patentamt
European Patent Office
Office européen des brevets

(11) **EP 3 495 817 B1**

(12) **EUROPEAN PATENT SPECIFICATION**

(45) Date of publication and mention
of the grant of the patent:
**16.10.2024 Bulletin 2024/42**

(21) Application number: **18193992.7**

(22) Date of filing: **11.02.2013**

(51) International Patent Classification (IPC):
**C12Q 1/6883** (2018.01)    **C12Q 1/6827** (2018.01)
**C12Q 1/6844** (2018.01)    **C12Q 1/6886** (2018.01)

(52) Cooperative Patent Classification (CPC):
(C-Sets available)
**C12Q 1/6883; C12Q 1/6827; C12Q 1/6844;**
**C12Q 1/6886;** C12Q 2600/156            (Cont.)

(54) **MOLECULAR DIAGNOSTIC SCREENING ASSAY**

MOLEKULARDIAGNOSTISCHER SCREENINGTEST

ESSAI DE CRIBLAGE DIAGNOSTIQUE MOLÉCULAIRE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **10.02.2012 US 201261597611 P**

(43) Date of publication of application:
**12.06.2019 Bulletin 2019/24**

(62) Document number(s) of the earlier application(s) in
accordance with Art. 76 EPC:
**13746127.3 / 2 823 303**

(73) Proprietor: **Bio-Rad Laboratories, Inc.**
**Hercules, CA 94547 (US)**

(72) Inventors:
• **WATSON, Andrew**
  **Bedford, MA 01730 (US)**
• **LINK, Darren, Roy**
  **Lexington, MA 02421 (US)**
• **OLSON, Jeffrey, Charles**
  **Chelmsford, MA 01824 (US)**
• **SAMUELS, Michael**
  **Windham, NH 03087 (US)**

(74) Representative: **Graham Watt & Co LLP**
**St. Botolph's House**
**7-9 St. Botolph's Road**
**Sevenoaks TN13 3AJ (GB)**

(56) References cited:
EP-A1- 1 829 964      WO-A1-99/28507
WO-A2-2006/042303   US-A1- 2011 159 499

• EIJK-VAN OS PETRA G C ET AL: "Multiplex Ligation-dependent Probe Amplification (MLPA(R)) for the detection of copy number variation in genomic sequences", METHODS IN MOLECULAR BIOLOGY, HUMANA PRESS, INC, US, vol. 688, 1 January 2011 (2011-01-01), pages 97 - 126, XP009184854, ISSN: 1940-6029
• Q. GUO ET AL: "Simultaneous Detection of Trisomies 13, 18, and 21 with Multiplex Ligation-Dependent Probe Amplification-Based Real-Time PCR", CLINICAL CHEMISTRY, vol. 56, no. 9, 1 September 2010 (2010-09-01), pages 1451 - 1459, XP055189889, ISSN: 0009-9147, DOI: 10.1373/clinchem.2010.146472
• CHIU ROSSA W K ET AL: "Noninvasive prenatal diagnosis of fetal chromosomal aneuploidy by massively parallel genomic sequencing of DNA in maternal plasma", PROCEEDINGS OF THE NATIONAL ACADEMY OF SCIENCES, NATIONAL ACADEMY OF SCIENCES, US, vol. 105, no. 51, 23 December 2008 (2008-12-23), pages 20458 - 20463, XP002620454, ISSN: 0027-8424, [retrieved on 20081210], DOI: 10.1073/PNAS.0810641105
• EIJK-VAN OS PETRA G C ET AL: "Multiplex Ligation-dependent Probe Amplification (MLPA(R)) for the detection of copy number variation in genomic sequences", METHODS IN MOLECULAR BIOLOGY; [METHODS IN MOLECULAR BIOLOGY; ISSN 1064-3745; VOL. 1310], HUMANA PR, US, vol. 688, 1 January 2011 (2011-01-01), pages 97 - 126, XP009184854, ISBN: 978-1-61779-291-5

**(Cont. next page)**

EP 3 495 817 B1

Remarks:
The complete document including Reference Table(s) and the Sequence Listing(s) can be downloaded from the EPO website

(52) Cooperative Patent Classification (CPC): (Cont.)

C-Sets
**C12Q 1/6827, C12Q 2525/155, C12Q 2525/161, C12Q 2563/159, C12Q 2563/179, C12Q 2565/629; C12Q 1/6844, C12Q 2521/319, C12Q 2521/501, C12Q 2525/155, C12Q 2545/101, C12Q 2563/159, C12Q 2563/179**

Remarks:

**Description**

Field of the Invention

The invention relates to a method for detecting aneuploidy, as defined in appended claims.

Background

Assays have been developed that rely on analyzing nucleic acid molecules from bodily fluids for the presence of abnormalities, thus leading to early diagnosis of certain conditions such as cancer or fetal aneuploidy. In a typical bodily fluid sample however, a majority of the nucleic acid is degraded, and any altered nucleic acids containing an abnormality of interest are present in small amounts (e.g., less than 1%) relative to a total amount of nucleic acids in the sample. This results in a failure to detect the small amount of abnormal nucleic acid.

Amplification based approaches (e.g., polymerase chain reaction (PCR), digital PCR, quantitative PCR) have previously been employed to attempt to detect these abnormalities. However, due to the stochastic nature of the amplification reaction, a population of molecules that is present in a small amount in the sample often is overlooked. In fact, if rare nucleic acid is not amplified in the first few rounds of amplification, it becomes increasingly unlikely that the rare event will ever be detected. Thus, the resulting biased post-amplification nucleic acid population does not represent the true condition of the sample from which it was obtained.

The advent of next generation sequencing methods, such as those commercially available from Roche (454 and SOLiD), Illumina/Solexa, Pacific Biosciences, and Life Technologies / Ion Torrent allow for the highly sensitive detection of the small population of abnormal nucleic acids in a sample, generally without the need for amplification of the nucleic acid in the sample. However, sequencing instruments are very expensive and these sequencing methods still require a large amount of data (for example, approximately 1,000,000 sequencing reads) to reliably identify an abnormal nucleic acid in a sample. Thus, sequencing is a very costly approach that still requires a significant amount of data in order to identify the presence of a population of abnormal nucleic acids in a sample.

Summary

Methods of the disclosure are able to identify an altered nucleic acid containing an abnormality of interest that is present in small amounts (e.g., less than 1%) relative to a total amount of nucleic acids in a sample. Methods of the invention are accomplished by designing a plurality of binders (e.g., 10 binders, 100 binders, 1,000 binders) against the nucleic acid that includes the abnormality of interest (i.e., the target nuclei acid). The binders each include a region that hybridizes to a different location on the target nucleic acid, and thus, all of the binders hybridize to the target nucleic acid at once. The target nucleic acid is separated from the sample and the hybridized binders are analyzed (e.g., PCR, digital PCR, qPCR, sequencing, etc.). This approach effectively increases the number of counts and confidence in the number of counts by a factor given by the number of binders against the target nucleic acid. For example, assuming only 1,000 genome equivalents of the target nucleic acid in the sample are available, and there are 1,000 binders, each of which binds to a different location on the target, there are now 1,000,000 target readouts, which is enough to identify a rare abnormal nucleic acid in a sample.

In certain aspects, methods of the disclosure involve obtaining a pool of nucleic acids from a sample, incubating the nucleic acids with first and second sets of binders, in which the first set binds uniquely to different regions of a target nucleic acid in the pool, the second set binds uniquely to different regions of a reference nucleic acid in the pool, and the first and second sets include different detectable labels, removing unbound binders, detecting the labels, and screening for a condition based upon results of the detecting step.

Detecting of the label may be accomplished by any analytical method known in the art. In certain embodiments, the detectable labels are barcode sequences and detecting the label includes sequencing the barcodes. In embodiments that using sequencing, screening for the condition may involve counting a number of barcodes from the first set, counting a number of barcodes from the second set, and determining whether a statistical difference exists between the number of barcodes from the first set and the number of barcodes from the second set.

In other embodiments, detecting the label may be accomplished by an amplification based technique, such as PCR, digital PCR, or qPCR. In specific embodiments, digital PCR is used to detect the labels. In these embodiments, after the removing step, the method further includes compartmentalizing bound binders of the first and second set into compartmentalized portions, the compartmentalized portions including, on average, either the first set of binders or the second set of binders, and amplifying binders in the compartmentalized portions. Compartmentalizing may involve diluting the sample such that it may be dispensed into different wells of a multi-well plate in a manner such that each well includes, on average, either the first set of binders or the second set of binders. Other exemplary compartmentalizing techniques are shown for example in, Griffiths et al. (U.S. patent number 7,968,287) and Link et al. (U.S. patent application number 2008/0014589).

In certain embodiments, the compartmentalizing involves forming droplets and the compartmentalized portions are the droplets. An exemplary method for forming droplets involves flowing a stream of sample fluid including the amplicons such that it intersects two opposing streams of flowing carrier fluid. The carrier fluid is immiscible with the sample fluid. Intersection of the sample fluid with the two opposing streams of flowing carrier fluid results in partitioning of the sample fluid into individual sample droplets. The carrier fluid may be any fluid that is immiscible with the sample fluid. An exemplary carrier fluid is oil, particularly, a fluorinated oil. In certain embodiments, the carrier fluid includes a surfactant, such as a fluorosurfactant. The droplets may be flowed through channels.

Generally, the binders of the first set include the same universal primer site and the binders of the second set include the same universal primer site, in which the primer sites of the first and second binders are different. Each compartmentalized portion further includes universal primers that bind the universal priming sites of the binders of the first set and universal primers that bind the universal priming sites of the binders of the second set. The compartmentalized portions further include probes that bind the detectable label of the first set of binders and probes that bind the detectable label of the second set of binders. An amplification reaction (e.g., PCR) is conducted in each compartmentalized portion, and the first probes are allowed to bind to the detectable label of the first set of binders, and the second probes are allowed to bind to the detectable label of the second set of binders. In such methods, the probes are optically labeled probes and detecting the label includes optically detecting the bound probes.

In such methods, screening for the condition may involve counting a number of compartmentalized portions including the first detectable label, counting a number of compartmentalized portions comprising the second detectable label, and determining whether a statistical difference exists between the number of compartmentalized portions comprising the first detectable label and the number of compartmentalized portions comprising the second detectable label.

The method of the invention is to screen for fetal aneuploidy. The fetal aneuploidy is trisomy 21 (Down syndrome). To look for fetal aneuploidy, one can use any maternal sample that may include fetal cell-free circulating nucleic acid. Exemplary samples include blood, plasma, or serum. In these embodiments, the target nucleic acid is nucleic acid of chromosome 21 and the first set of binders binds to the nucleic acid of chromosome 21 in the pool. The second set of binders binds nucleic acid of a reference chromosome in the pool.

In certain embodiments, the detectable labels are barcode sequences and detecting the label includes sequencing the barcodes. In embodiments that using sequencing, screening for the condition may involve counting a number of barcodes from the first set, counting a number of barcodes from the second set, and determining whether a statistical difference exists between the number of barcodes from the first set and the number of barcodes from the second set. In other embodiments, detecting the label may be accomplished by an amplification based technique, such as PCR, digital PCR, or qPCR. In specific embodiments, digital PCR is used to detect the labels. In such methods, screening for the condition may involve counting a number of compartmentalized portions including the first detectable label, counting a number of compartmentalized portions comprising the second detectable label, and determining whether a statistical difference exists between the number of compartmentalized portions comprising the first detectable label and the number of compartmentalized portions comprising the second detectable label.

In other embodiments, methods of the invention are used to screen a subject for cancer generally. In these embodiments, the first set of binders binds genomic regions of the nucleic acids associated with known mutations involved in different cancers and the second set of binders binds genomic regions of the nucleic acids that are not mutated. In embodiments that using sequencing, screening for the condition may involve counting a number of barcodes from the first set, counting a number of barcodes from the second set, and determining whether a statistical difference exists between the number of barcodes from the first set and the number of barcodes from the second set. In other embodiments, detecting the label may be accomplished by an amplification based technique, such as PCR, digital PCR, or qPCR. In specific embodiments, digital PCR is used to detect the labels. In such methods, screening for the condition may involve counting a number of compartmentalized portions including the first detectable label, counting a number of compartmentalized portions comprising the second detectable label, and determining whether a statistical difference exists between the number of compartmentalized portions comprising the first detectable label and the number of compartmentalized portions comprising the second detectable label.

Another aspect of the disclosure provides methods for screening for a condition in a subject, that involve obtaining a pool of different nucleic acid from a sample, compartmentalizing the pool of nucleic acids into compartmentalized portions, the compartmentalized portions including, on average, either a first set of binders or a second set of binders, wherein the first and second sets comprise different detectable labels and the first and second sets bind to different nucleic acids in the pool, amplifying only binders in the compartmentalized portions that bound to the nucleic acid, detecting the labels, and screening for a condition based upon results of the detecting step.

Brief Description of the Drawings

Figures 1A-B show one embodiment of first and second binders of the invention.

Figures 2A-B show another embodiments of binders of the invention.

Figures 3A-B shows the binders of Figures 2A-B in circularized form.

Figures 4A-B illustrate how circularized binders are amplified while non-circularized binders are not amplified.

Figures 5A-B shows an exemplary embodiment of a device for droplet formation.

Figure 6 shows an embodiment of using methods of the invention to screen for fetal aneuploidy, particularly trisomy 21 (Down syndrome).

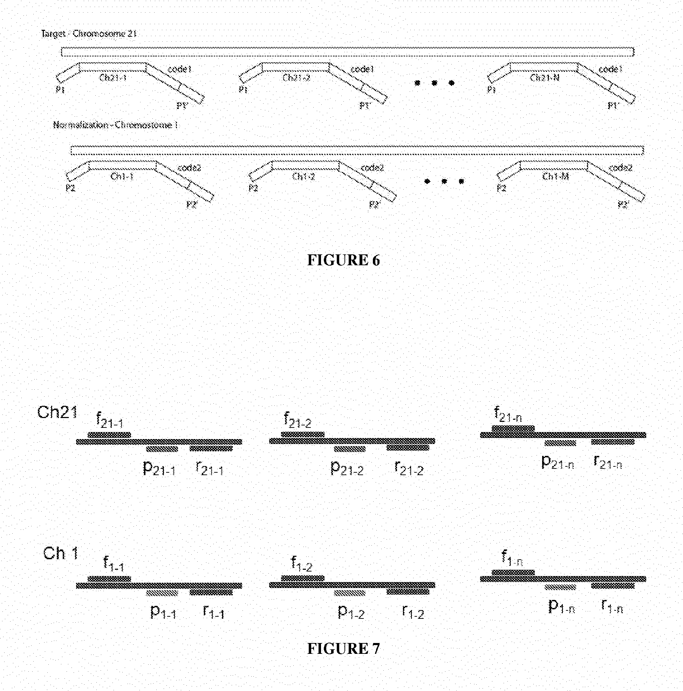

Figure 7 shows an embodiment of a multiple TAQMAN (hydrolysis probes, Invitrogen, Inc.) assay with assay specific probes. A first assay is conducted on chromosome 21. $p_{21-1}$ to $p_{21-n}$ are all different target sequences on chromosome 21, but the same fluorophore. $f_{21-1}$ to $f_{21-n}$ are the forward PCR primers and $r_{21-1}$ to $r_{21-n}$ are the reverse PCR primers. Another assay is conducted on a normalization chromosome, in this case, chromosome 1. However, the second assay uses a different set of probes and a different color. $p_{1-1}$ to $p_{1-n}$ are all different target sequences on chromosome 1, but the same fluorophore. $f_{1-1}$ to $f_{1-n}$ are the forward PCR primers and $r_{1-1}$ to $r_{1-n}$ are the reverse PCR primers.

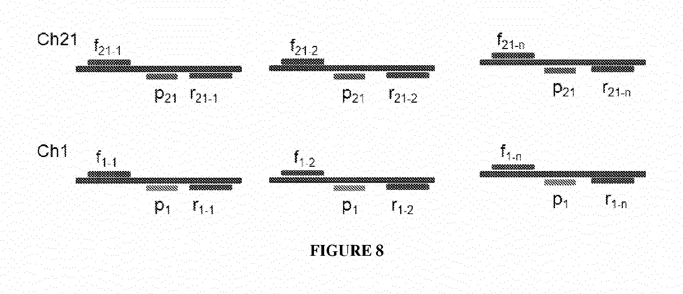

Figure 8 shows an embodiment of an assay that uses a set of probes that hybridize to multiple regions in the genome. For this embodiment, a set of primers is selected that flank a common probe site on chromosome 21. Additionally, a set of primers is selected that flank a different common probe sight on chromosome 1. As compared to the embodiment described in Figure 7, the assay in this embodiment reduces the number of different probes that have to be in the mixture, thereby decreasing the amount of background fluorescence. In this embodiment, the specificity may primarily or exclusively come from the primers as the probes may hybridize to many locations throughout the genome.

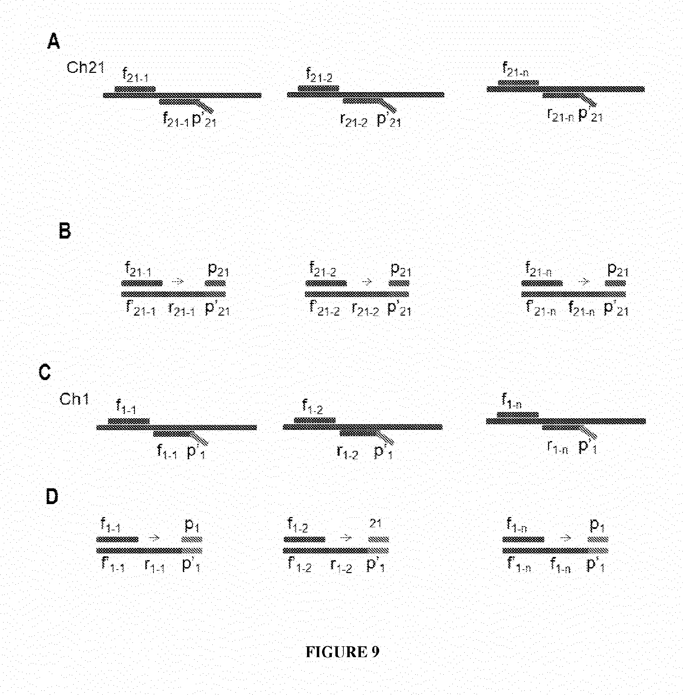

Figure 9 panels A-D show an embodiment of an assay that uses multiple primers to each of chromosome 21 and chromosome 1, in which the primers have a chromosome specific probe location on the primer. Panel A shows chromosome 21 with a set of forward primers ($f_{21-1}$, $f_{21-2}$, ... $f_{21-n}$) and a set of reverse primers ($r_{21-1}$, $r_{21-2}$, ... $r_{21-n}$). Each of the reverse primers has a universal probe annealing site ($p'_{21}$) at the end. Panel B shows PCR product with probe annealing site on the end. $p_{21}$ is a universal probe that hybridizes to all PCR amplicons for chromosome 21. $f_{21-1}$ to $f_{21-n}$ are the forward PCR primers and $r_{21-1}$ to $r_{21-n}$ are the reverse PCR primers. Panel C shows chromosome 1 with a set of forward primers ($f_{1-1}$, $f_{1-2}$, ... $f_{1-n}$) and a set of reverse primers ($r_{1-1}$, $r_{1-2}$, ... $r_{1-n}$). Each of the reverse primers has a universal probe annealing site ($p'_{1}$) at the end. Panel D shows PCR product with probe annealing site on the end. $p_1$ is a universal probe that hybridizes to all PCR amplicons for chromosome 1. $f_{1-1}$ to $f_{1-n}$ are the forward PCR primers and $r_{1-1}$ to $r_{1-n}$ are the reverse PCR primers.

Figure 10 shows a number of TAGS. Each TAG is constructed from an 'a' and a 'b' portion such that a complete TAG is constructed by ligation or a fill and ligate process. It is then likely to be unnecessary to remove unbound tags. However, in some cases it may be desirable to remove unbound tags, in which case they can be removed by using 3' & 5' exonuclease and protected ends on the half TAGs.

In scenarios where there is a limiting amount of starting DNA, multiple TAGS can be generated from the same starting target by melting off of the complete TAG and then annealing 'a' and 'b' fragments. This would be a linear amplification of the number of complete TAGS constructed.

Figure 11 shows a scatter plot for the reaction DMDi3 Hyb A2 + DMDi3 Hyb B2 .

Figure 12 shows a scatter plot for the reaction DMDe8 Hyb A2 + DMDe8 Hyb B2

Figure 13 shows a scatter plot for the reaction DMDe8 Hyb A2 + DMDe8 Hyb B2 tile + DMDi3 Hyb A2 + DMDi3 Hyb B2.

Detailed Description

The invention generally relates to method for screening for a condition in a subject. In certain embodiments, methods of the invention involve obtaining a pool of nucleic acids from a sample, incubating the nucleic acids with first and second sets of binders, in which the first set binds uniquely to different regions of a target nucleic acid in the pool, the second set binds uniquely to different regions of a reference nucleic acid in the pool, and the first and second sets include different detectable labels, removing unbound binders, detecting the labels, and screening for a condition based upon results of the detecting step. It is important to note that in methods of the invention, the binders, and not the nucleic acid, is analyzed for the purpose of screening for a condition. Methods and tools for detecting target sequences are i.a. provided in van Eijk et al., Meth. mol. Biology, 688, pp.97-126, 2011; WO9928507 A1; Guo et al., Clin. Chem. 56, pp.1451-1459, 2010; US2011159499 A1; EP18259964 and WO2006042303.

Nucleic Acids

Nucleic acid is generally is acquired from a sample or a subject. Target molecules for labeling and/or detection according to the methods of the invention include, but are not limited to, genetic and proteomic material, such as DNA, genomic DNA, RNA, expressed RNA and/or chromosome(s). Methods of the invention are applicable to DNA from whole cells or to portions of genetic or proteomic material obtained from one or more cells. For a subject, the sample may be obtained in any clinically acceptable manner, and the nucleic acid templates are extracted from the sample by methods known in the art. Nucleic acid templates can be obtained as described in U.S. Patent Application Publication Number US2002/0190663 A1, published Oct. 9, 2003. Generally, nucleic acid can be extracted from a biological sample by a variety of techniques such as those described by Maniatis, et al. (Molecular Cloning: A Laboratory Manual, Cold Spring Harbor, N.Y., pp. 280-281, 1982). Nucleic acid templates include deoxyribonucleic acid (DNA) and/or ribonucleic acid (RNA). Nucleic acid templates can be synthetic or derived from naturally occurring sources. In one embodiment, nucleic acid templates are isolated from a biological sample containing a variety of other components, such as proteins, lipids and non-template nucleic acids. Nucleic acid templates can be obtained from any cellular material, obtained from an animal, plant, bacterium, fungus, or any other cellular organism. Biological samples for use in the present invention include viral particles or preparations. Nucleic acid templates can be obtained directly from an organism or from a biological sample obtained from an organism, e.g., from blood, urine, cerebrospinal fluid, seminal fluid, saliva, sputum, stool and tissue. In a particular embodiment, nucleic acid is obtained from fresh frozen plasma (FFP). Any tissue or body fluid specimen may be used as a source for nucleic acid for use in the invention. Nucleic acid templates can also be isolated from cultured cells, such as a primary cell culture or a cell line. The cells or tissues from which template nucleic acids are obtained can be infected with a virus or other intracellular pathogen. A sample can also be total RNA extracted from a biological specimen, a cDNA library, viral, or genomic DNA.

Generally, nucleic acid obtained from biological samples is fragmented to produce suitable fragments for analysis. An advantage of methods of the invention is that they can be performed on nucleic acids that have not been fragmented.

However, in certain embodiments, nucleic acids are fragmented prior to performing methods of the invention. In one embodiment, nucleic acid from a biological sample is fragmented by sonication. Generally, individual nucleic acid template molecules can be from about 5 bases to about 20 kb.

A biological sample as described herein may be homogenized or fractionated in the presence of a detergent or surfactant. The concentration of the detergent in the buffer may be about 0.05% to about 10.0%. The concentration of the detergent can be up to an amount where the detergent remains soluble in the solution. In a preferred embodiment, the concentration of the detergent is between 0.1% to about 2%. The detergent, particularly a mild one that is nondenaturing, can act to solubilize the sample. Detergents may be ionic or nonionic. Examples of nonionic detergents include triton, such as the Triton® X series (Triton® X-100 t-Oct-C6H4-(OCH2-CH2)xOH, x=9-10, Triton® X-100R, Triton® X-114 x=7-8), octyl glucoside, polyoxyethylene(9)dodecyl ether, digitonin, IGEPAL® CA630 octylphenyl polyethylene glycol, n-octyl-beta-D-glucopyranoside (betaOG), n-dodecyl-beta, Tween® 20 polyethylene glycol sorbitan monolaurate, Tween® 80 polyethylene glycol sorbitan monooleate, polidocanol, n-dodecyl beta-D-maltoside (DDM), NP-40 nonylphenyl polyethylene glycol, C12E8 (octaethylene glycol n-dodecyl monoether), hexaethyleneglycol mono-n-tetradecyl ether (C14EO6), octyl-beta-thioglucopyranoside (octyl thioglucoside, OTG), Emulgen, and polyoxyethylene 10 lauryl ether (C12E10). Examples of ionic detergents (anionic or cationic) include deoxycholate, sodium dodecyl sulfate (SDS), N-lauroylsarcosine, and cetyltrimethylammoniumbromide (CTAB). A zwitterionic reagent may also be used in the purification schemes of the present invention, such as Chaps, zwitterion 3-14, and 3-[(3-cholamidopropyl)dimethylammonio]-1-propanesulf-onate. It is contemplated also that urea may be added with or without another detergent or surfactant.

Lysis or homogenization solutions may further contain other agents, such as reducing agents. Examples of such reducing agents include dithiothreitol (DTT), .beta.-mercaptoethanol, DTE, GSH, cysteine, cysteamine, tricarboxyethyl phosphine (TCEP), or salts of sulfurous acid.

Once obtained, the nucleic acid is denatured by any method known in the art to produce single stranded nucleic acid templates and a pair of first and second oligonucleotides is hybridized to the single stranded nucleic acid template such that the first and second oligonucleotides flank a target region on the template.

Nucleic acid Binders

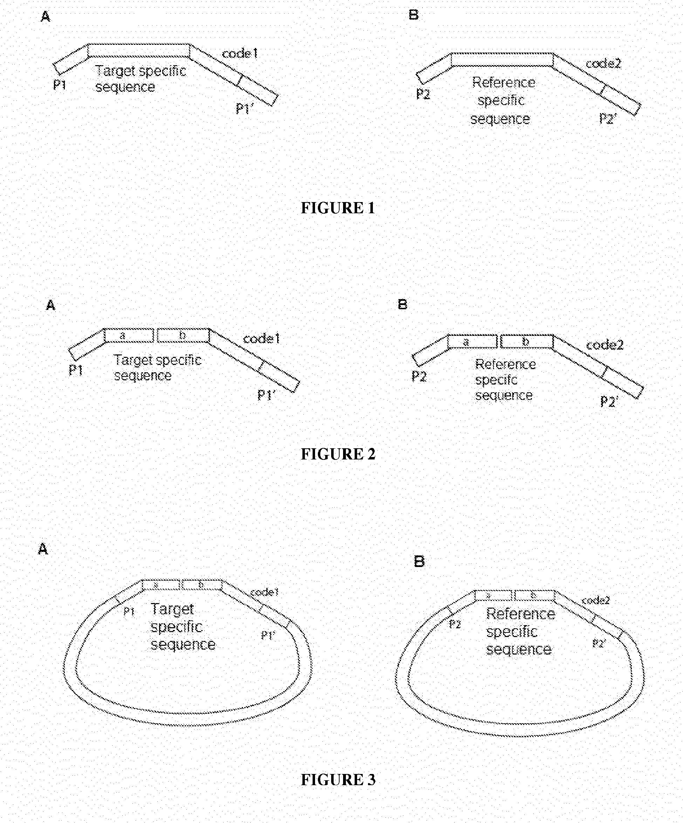

Methods of the invention involve using first and second sets of binders. In certain embodiments, the first set of binders generally have the structure shown in Figure 1A and the second set of binders have a structure as shown in Figure 1B. Each binder includes a pair of universal forward and reverse primer sites (P1 and P1' and P2 and P2'). All of the binders of the first set include the same set of universal primer sites. All of the binders of the second set include the same set of universal primer sites. The primers sites of the first set are different than the primer sites of the second set, i.e., P1 and P1' are different than P2 and P2'. Each of the binders includes a detectable label code site. All of the binders of the first set include the same detectable label code site. All of the binders of the second set include the same detectable label code site. The detectable label code site of the first set is different than the detectable label code site of the second set.

Each of the binders in the first set includes a target sequence portion. The target sequence portion for each of the binders of the first set is a sequence that binds to a region of the target nucleic acid. However, the target sequence portion of each binder of the first set is different, so that each binder of the first set may bind to a different location of the target nucleic acid.

Each of the binders in the second set includes a reference sequence portion. The reference sequence portion for each of the binders of the second set is a sequence that binds to a region of the reference nucleic acid. However, the reference sequence portion of each binder of the second set is different, so that each binder of the second set may bind to a different location of the reference nucleic acid.

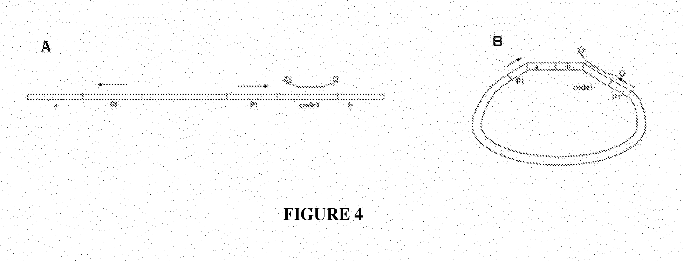

In certain embodiments, the first set of binders generally have the structure shown in Figure 2A and the second set of binders have a structure as shown in Figure 2B. In this embodiments, the first and second binders are constructed from an "a" portion and a "b" portion such that a complete binder is constructed by ligation or a fill and ligate process. This type of structure avoids the need to remove unbound binders, because only fully formed binders (i.e., those with an "a" and "b") portion can be analyzed. Figures 3A-B show complete binders. If the binder is constructed such that the digital PCR annealing region is only amplified by the primers when a circle is constructed, then a probe, such as a Taqman probe, would not be hydrolyzed exponentially as the PCR reaction proceeded. This is illustrated in Figures 4A-B.

The type of detectable label code site in the first and second binders will depend on the binder detection technique to be employed. If the binder detection technique is sequencing, the detectable label code site will be a barcode sequence. In these embodiments, all of the binders of the first set will have the same barcode sequence and all of the binders of the second set will have the same barcode sequence. The barcode sequence of the first set of binders is different than the barcode sequence of the second set of binders.

If the binder detection technique is probe hybridization, the detectable label code site will be a sequence that hybridizes with the probe. In these embodiments, all of the binders of the first set will hybridize with a first probe and all of the binders of the second set will hybridize with a second probe. The first and second probe are different and include different labels.

Methods of synthesizing oligonucleotide are known in the art. See, e.g., Sambrook et al. (DNA microarray: A Molecular Cloning Manual, Cold Spring Harbor, N.Y., 2003) or Maniatis, et al. (Molecular Cloning: A Laboratory Manual, Cold Spring Harbor, N.Y., 1982), the contents of each of which are incorporated by reference herein in their entirety. Suitable methods for synthesizing oligonucleotide are also described in Caruthers (Science 230:281-285, 1985),. Oligonucleotides can also be obtained from commercial sources such as Operon Technologies, Amersham Pharmacia Biotech, Sigma, and Life Technologies. The oligonucleotides can have an identical melting temperature. The lengths of the oligonucleotides can be extended or shortened at the 5' end or the 3' end to produce oligonucleotides with desired melting temperatures. Also, the annealing position of each oligonucleotide can be designed such that the sequence and length of the probe yield the desired melting temperature. The simplest equation for determining the melting temperature of probes smaller than 25 base pairs is the Wallace Rule (Td=2(A+T)+4(G+C)). Computer programs can also be used to design oligonucleotides, including but not limited to Array Designer Software (Arrayit Inc.), Oligonucleotide Probe Sequence Design Software for Genetic Analysis (Olympus Optical Co.), NetPrimer, and DNAsis from Hitachi Software Engineering. The TM (melting temperature) of each probe is calculated using software programs such as Oligo Design, available from Invitrogen Corp.

In certain embodiments, reaction conditions of high stringency are used to ensure great specificity between the probes and the code sites on the binders. Nucleic acid hybridization may be affected by such conditions as salt concentration, temperature, or organic solvents, in addition to base composition, length of complementary strands, and number of nucleotide base mismatches between hybridizing nucleic acids, as is readily appreciated by those skilled in the art. Stringency of hybridization reactions is readily determinable by one of ordinary skill in the art, and generally is an empirical calculation dependent upon sequence length, washing temperature, and salt concentration. In general, longer sequences require higher temperatures for proper annealing, while shorter sequences need lower temperatures. Hybridization generally depends on the ability of denatured DNA to re-anneal when complementary strands are present in an environment below its melting temperature. The higher the degree of desired homology between the sequence and hybridizable sequence, the higher the relative temperature that can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

Stringent conditions or high stringency conditions typically: (1) employ low ionic strength and high temperature for washing, for example 0.015 M sodium chloride/0.0015 M sodium citrate/0.1% sodium dodecyl sulfate at 50°C.; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42°C.; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 .mu.g/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

In other embodiments, reaction conditions of moderate stringency are used for hybridization of the first and second oligonucleotides to binding regions on the template nucleic acid. Moderately stringent conditions may be identified as described by Sambrook et al., Molecular Cloning: A Laboratory Manual, New York: Cold Spring Harbor Press, 1989 (the contents of which are incorporated by reference herein in their entirety and include the use of washing solution and hybridization conditions (e.g., temperature, ionic strength and % SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37°C to 50°C. The skilled artisan will recognize how to adjust the temperature, ionic strength, etc. as necessary to accommodate factors such as sequence length and the like.

Oligonucleotides suitable for use in the present invention include those formed from nucleic acids, such as RNA and/or DNA, nucleic acid analogs, locked nucleic acids, modified nucleic acids, and chimeric probes of a mixed class including a nucleic acid with another organic component such as peptide nucleic acids. Exemplary nucleotide analogs include phosphate esters of deoxyadenosine, deoxycytidine, deoxyguanosine, deoxythymidine, adenosine, cytidine, guanosine, and uridine. Other examples of non-natural nucleotides include a xanthine or hypoxanthine; 5-bromouracil, 2-aminopurine, deoxyinosine, or methylated cytosine, such as 5-methylcytosine, and N4-methoxydeoxycytosine. Also included are bases of polynucleotide mimetics, such as methylated nucleic acids, e.g., 2'-O-methRNA, peptide nucleic acids, modified peptide nucleic acids, and any other structural moiety that can act substantially like a nucleotide or base, for example, by exhibiting base-complementarity with one or more bases that occur in DNA or RNA.

The length of the oligonucleotide probe is not critical, as long as the oligonucleotides are capable of hybridizing to the code sites of the binders. In fact, oligonucleotides may be of any length. For example, oligonucleotides may be as few as 5 nucleotides, or as much as 5000 nucleotides. Exemplary oligonucleotides are 5-mers, 10-mers, 15-mers, 20-mers, 25-mers, 50-mers, 100-mers, 200-mers, 500-mers, 1000-mers, 3000-mers, or 5000-mers. Methods for determining an optimal oligonucleotides length are known in the art. See, e.g., Shuber (U.S. patent number 5,888,778). The first and second oligonucleotides do not have to be of the same length. In certain embodiments, the first and second oligonucleotides are the same length, while in other embodiments, the first and second oligonucleotides are of different lengths.

The reaction time will depend on the different factors discussed above, e.g., stringency conditions, probe length, probe design, etc.

Generally, the probes will include a detectable label that is directly or indirectly detectable. Preferred labels include optically-detectable labels, such as fluorescent labels. Examples of fluorescent labels include, but are not limited to, 4-acetamido-4'-isothiocyanatostilbene-2,2'disulfonic acid; acridine and derivatives: acridine, acridine isothiocyanate; 5-(2'-aminoethyl)aminonaphthalene-1-sulfonic acid (EDANS); 4-amino-N-[3-vinylsulfonyl)phenyl]naphthalimide-3,5 disulfonate; N-(4-anilino-1-naphthyl)maleimide; anthranilamide; BODIPY; Brilliant Yellow; coumarin and derivatives; coumarin, 7-amino-4-methylcoumarin (AMC, Coumarin 120), 7-amino-4-trifluoromethylcoular in (Coumaran 151); cyanine dyes; cyanosine; 4',6-diaminidino-2-phenylindole (DAPI); 5'5"-dibromopyrogallol-sulfonaphthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]naphthalene-1-sulfonyl chloride (DNS, dansylchloride); 4-dimethylaminophenylazophenyl-4'-isothiocyanate (DABITC); eosin and derivatives; eosin, eosin isothiocyanate, erythrosin and derivatives; erythrosin B, erythrosin, isothiocyanate; ethidium; fluorescein and derivatives; 5-carboxyfluorescein (FAM), 5-(4,6-dichlorotriazin-2-yl)aminofluorescein (DTAF), 2',7'-dimethoxy-4'5'-dichloro-6-carboxyfluorescein, fluorescein, fluorescein isothiocyanate, QFITC, (XRITC); fluorescamine; IR144; IR1446; Malachite Green isothiocyanate; 4-methylumbelliferoneortho cresolphthalein; nitrotyrosine; pararosaniline; Phenol Red; B-phycoerythrin; o-phthaldialdehyde; pyrene and derivatives: pyrene, pyrene butyrate, succinimidyl 1-pyrene; butyrate quantum dots; Reactive Red 4 (Cibacron.TM. Brilliant Red 3B-A) rhodamine and derivatives: 6-carboxy-X-rhodamine (ROX), 6-carboxyrhodamine (R6G), lissamine rhodamine B sulfonyl chloride rhodamine (Rhod), rhodamine B, rhodamine 123, rhodamine X isothiocyanate, sulforhodamine B, sulforhodamine 101, sulfonyl chloride derivative of sulforhodamine 101 (Texas Red); N,N,N',N'tetramethyl-6-carboxyrhodamine (TAMRA); tetramethyl rhodamine; tetramethyl rhodamine isothiocyanate (TRITC); riboflavin; rosolic acid; terbium chelate derivatives; Cy3; Cy5; Cy5.5; Cy7; IRD 700; IRD 800; La Jolta Blue; phthalo cyanine; and naphthalo cyanine. Preferred fluorescent labels are cyanine-3 and cyanine-5. Labels other than fluorescent labels are contemplated by the invention, including other optically-detectable labels.

Incubation

The pool of nucleic acids is incubated with first and second sets of binders under conditions that allow the first set of binders to bind to target nucleic acids in the pool and the second set of binders to bind the reference nucleic acids in the pool.

In certain embodiments, reaction conditions of high stringency are used to ensure great specificity between the probes and the code sites on the binders. Nucleic acid hybridization may be affected by such conditions as salt concentration, temperature, or organic solvents, in addition to base composition, length of complementary strands, and number of nucleotide base mismatches between hybridizing nucleic acids, as is readily appreciated by those skilled in the art. Stringency of hybridization reactions is readily determinable by one of ordinary skill in the art, and generally is an empirical calculation dependent upon sequence length, washing temperature, and salt concentration. In general, longer sequences require higher temperatures for proper annealing, while shorter sequences need lower temperatures. Hybridization generally depends on the ability of denatured DNA to re-anneal when complementary strands are present in an environment below its melting temperature. The higher the degree of desired homology between the sequence and hybridizable sequence, the higher the relative temperature that can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

Stringent conditions or high stringency conditions typically: (1) employ low ionic strength and high temperature for washing, for example 0.015 M sodium chloride/0.0015 M sodium citrate/0.1% sodium dodecyl sulfate at 50°C.; (2) employ during hybridization a denaturing agent, such as formamide, for example, 50% (v/v) formamide with 0.1% bovine serum albumin/0.1% Ficoll/0.1% polyvinylpyrrolidone/50 mM sodium phosphate buffer at pH 6.5 with 750 mM sodium chloride, 75 mM sodium citrate at 42°C.; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 .mu.g/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

In other embodiments, reaction conditions of moderate stringency are used for hybridization of the first and second oligonucleotides to binding regions on the template nucleic acid. Moderately stringent conditions may be identified as described by Sambrook et al., Molecular Cloning: A Laboratory Manual, New York: Cold Spring Harbor Press, 1989 (the contents of which are incorporated by reference herein in their entirety and include the use of washing solution and hybridization conditions (e.g., temperature, ionic strength and % SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37°C to 50°C. The skilled artisan will recognize how to adjust the temperature, ionic strength, etc. as necessary to accommodate factors such as sequence length and the like.

Oligonucleotides suitable for use in the present invention include those formed from nucleic acids, such as RNA and/or DNA, nucleic acid analogs, locked nucleic acids, modified nucleic acids, and chimeric probes of a mixed class including a nucleic acid with another organic component such as peptide nucleic acids. Exemplary nucleotide analogs include phosphate esters of deoxyadenosine, deoxycytidine, deoxyguanosine, deoxythymidine, adenosine, cytidine, guanosine, and uridine. Other examples of non-natural nucleotides include a xanthine or hypoxanthine; 5-bromouracil, 2-aminopurine, deoxyinosine, or methylated cytosine, such as 5-methylcytosine, and N4-methoxydeoxycytosine. Also included are bases of polynucleotide mimetics, such as methylated nucleic acids, e.g., 2'-O-methRNA, peptide nucleic acids, modified peptide nucleic acids, and any other structural moiety that can act substantially like a nucleotide or base, for example, by exhibiting base-complementarity with one or more bases that occur in DNA or RNA.

The reaction time will depend on the different factors discussed above, e.g., stringency conditions, probe length, probe design, etc.

Removing unbound binders

In certain embodiments, it is important to remove unbound binders. However, this is an optional step based upon the type of binders used with methods of the invention. As discussed above, binders can be constructed such that a removal step is not necessary and methods of the invention can be conducted with or without the removing step, i.e., this is an optional step.

Any method known in the art may be used for removing unbound binders. For example, the binders can be RNA binders and unbound binders can be removed by exonuclease digestion. Alternatively, the nucleic acid in the sample can be modified with a biotin tag prior to being incubated with the first and second binders. The incubated mixture can be exposed to a streptavidin coated surface, such as magnetic beads such that the nucleic acid in the sample hybridized with the binders binds to the streptavidin coated surface. A magnetic field can then be used to separate the unbound binders from nucleic acid having bound binders. Methods of modifying nucleic acids with biotin and then attaching to a streptavidin coated surface are known in the art, see for example, Lapidus et al. (U.S. patent number 7,169,560), Lapidus et al. (U.S. patent application number 2009/0191565), Quake et al. (U.S. patent number 6,818,395), Harris (U.S. patent number 7,282,337), and Quake et al. (U.S. patent application number 2002/0164629).

Various other attachment methods can be used to anchor or immobilize the nucleic acid molecule to the surface of a substrate. The immobilization can be achieved through direct or indirect bonding to the surface. The bonding can be by covalent linkage. See, Joos et al., Analytical Biochemistry 247:96-101, 1997; Oroskar et al., Clin. Chem. 42:1547-1555, 1996; and Khandjian, Mol. Bio. Rep. 11:107-115, 1986. An example of an attachment is direct amine bonding of a terminal nucleotide of the template or the 5' end of the primer to an epoxide integrated on the surface. The bonding also can be through non-covalent linkage. For example, biotin-streptavidin (Taylor et al., J. Phys. D. Appl. Phys. 24:1443, 1991) and digoxigenin with anti-digoxigenin (Smith et al., Science 253:1122, 1992) are common tools for anchoring nucleic acids to surfaces and parallels.

Sequencing detection methods

In certain embodiments, sequencing is used to detect the code site. Sequencing may be by any method known in the art. DNA sequencing techniques include classic dideoxy sequencing reactions (Sanger method) using labeled terminators or primers and gel separation in slab or capillary, sequencing by synthesis using reversibly terminated labeled nucleotides, pyrosequencing, 454 sequencing, allele specific hybridization to a library of labeled oligonucleotide probes, sequencing by synthesis using allele specific hybridization to a library of labeled clones that is followed by ligation, real time monitoring of the incorporation of labeled nucleotides during a polymerization step, polony sequencing, and SOLiD sequencing. Sequencing of separated molecules has more recently been demonstrated by sequential or single extension reactions using polymerases or ligases as well as by single or sequential differential hybridizations with libraries of probes.

A sequencing technique that can be used in the methods of the provided invention includes, for example, Helicos True Single Molecule Sequencing (tSMS) (Harris T. D. et al. (2008) Science 320:106-109). In the tSMS technique, a DNA sample is cleaved into strands of approximately 100 to 200 nucleotides, and a polyA sequence is added to the 3' end of each DNA strand. Each strand is labeled by the addition of a fluorescently labeled adenosine nucleotide. The DNA strands are then hybridized to a flow cell, which contains millions of oligo-T capture sites that are immobilized to the flow cell surface. The templates can be at a density of about 100 million templates/cm2. The flow cell is then loaded into an instrument, e.g., HeliScope.TM. sequencer, and a laser illuminates the surface of the flow cell, revealing the position of each template. A CCD camera can map the position of the templates on the flow cell surface. The template fluorescent label is then cleaved and washed away. The sequencing reaction begins by introducing a DNA polymerase and a fluorescently labeled nucleotide. The oligo-T nucleic acid serves as a primer. The polymerase incorporates the labeled nucleotides to the primer in a template directed manner. The polymerase and unincorporated nucleotides are removed. The templates that have directed incorporation of the fluorescently labeled nucleotide are detected by imaging the flow cell surface. After imaging, a cleavage step removes the fluorescent label, and the process is repeated with other fluorescently labeled nucleotides until the desired read length is achieved. Sequence information is collected with each nucleotide addition step. Further description of tSMS is shown for example in Lapidus et al. (U.S. patent number 7,169,560), Lapidus et al. (U.S. patent application number 2009/0191565), Quake et al. (U.S. patent number 6,818,395), Harris (U.S. patent number 7,282,337), Quake et al. (U.S. patent application number 2002/0164629), and Braslavsky, et al., PNAS (USA), 100: 3960-3964 (2003).

Another example of a DNA sequencing technique that can be used in the methods of the provided invention is 454 sequencing (Roche) (Margulies, M et al. 2005, Nature, 437, 376-380). 454 sequencing involves two steps. In the first step, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to DNA capture beads, e.g., streptavidin-coated beads using, e.g., Adaptor B, which contains 5'-biotin tag. The fragments attached to the beads are PCR amplified within droplets of an oil-water emulsion. The result is multiple copies of clonally amplified DNA fragments on each bead. In the second step, the beads are captured in wells (pico-liter sized). Pyrosequencing is performed on each DNA fragment in parallel. Addition of one or more nucleotides generates a light signal that is recorded by a CCD camera in a sequencing instrument. The signal strength is proportional to the number of nucleotides incorporated. Pyrosequencing makes use of pyrophosphate (PPi) which is released upon nucleotide addition. PPi is converted to ATP by ATP sulfurylase in the presence of adenosine 5' phosphosulfate. Luciferase uses ATP to convert luciferin to oxyluciferin, and this reaction generates light that is detected and analyzed.

Another example of a DNA sequencing technique that can be used in the methods of the provided invention is SOLiD technology (Applied Biosystems). In SOLiD sequencing, genomic DNA is sheared into fragments, and adaptors are attached to the 5' and 3' ends of the fragments to generate a fragment library. Alternatively, internal adaptors can be introduced by ligating adaptors to the 5' and 3' ends of the fragments, circularizing the fragments, digesting the circularized fragment to generate an internal adaptor, and attaching adaptors to the 5' and 3' ends of the resulting fragments to generate a mate-paired library. Next, clonal bead populations are prepared in microreactors containing beads, primers, template, and PCR components. Following PCR, the templates are denatured and beads are enriched to separate the beads with extended templates. Templates on the selected beads are subjected to a 3' modification that permits bonding to a glass slide. The sequence can be determined by sequential hybridization and ligation of partially random oligonucleotides with a central determined base (or pair of bases) that is identified by a specific fluorophore. After a color is recorded, the ligated oligonucleotide is cleaved and removed and the process is then repeated.

Another example of a DNA sequencing technique that can be used in the methods of the provided invention is Ion Torrent sequencing (U.S. patent application numbers 2009/0026082, 2009/0127589, 2010/0035252, 2010/0137143, 2010/0188073, 2010/0197507, 2010/0282617, 2010/0300559), 2010/0300895, 2010/0301398, and 2010/0304982). In Ion Torrent sequencing, DNA is sheared into fragments of approximately 300-800 base pairs, and the fragments are blunt ended. Oligonucleotide adaptors are then ligated to the ends of the fragments. The adaptors serve as primers for amplification and sequencing of the fragments. The fragments can be attached to a surface and is attached at a resolution such that the fragments are individually resolvable. Addition of one or more nucleotides releases a proton (H+), which signal detected and recorded in a sequencing instrument. The signal strength is proportional to the number of nucleotides incorporated.

Another example of a sequencing technology that can be used in the methods of the provided invention is Illumina sequencing. Illumina sequencing is based on the amplification of DNA on a solid surface using fold-back PCR and anchored primers. Genomic DNA is fragmented, and adapters are added to the 5' and 3' ends of the fragments. DNA fragments that are attached to the surface of flow cell channels are extended and bridge amplified. The fragments become double stranded, and the double stranded molecules are denatured. Multiple cycles of the solid-phase amplification followed by denaturation can create several million clusters of approximately 1,000 copies of single-stranded DNA molecules of the same template in each channel of the flow cell. Primers, DNA polymerase and four fluorophore-labeled, reversibly terminating nucleotides are used to perform sequential sequencing. After nucleotide incorporation, a laser is used to excite the fluorophores, and an image is captured and the identity of the first base is recorded. The 3' terminators and fluorophores from each incorporated base are removed and the incorporation, detection and identification steps are repeated.

Another example of a sequencing technology that can be used in the methods of the provided invention includes the single molecule, real-time (SMRT) technology of Pacific Biosciences. In SMRT, each of the four DNA bases is attached to one of four different fluorescent dyes. These dyes are phospholinked. A single DNA polymerase is immobilized with a single molecule of template single stranded DNA at the bottom of a zero-mode waveguide (ZMW). A ZMW is a confinement structure which enables observation of incorporation of a single nucleotide by DNA polymerase against the background of fluorescent nucleotides that rapidly diffuse in an out of the ZMW (in microseconds). It takes several milliseconds to incorporate a nucleotide into a growing strand. During this time, the fluorescent label is excited and produces a fluorescent signal, and the fluorescent tag is cleaved off. Detection of the corresponding fluorescence of the dye indicates which base was incorporated. The process is repeated.

Another example of a sequencing technique that can be used in the methods of the provided invention is nanopore sequencing (Soni G V and Meller A. (2007) Clin Chem 53: 1996-2001). A nanopore is a small hole, of the order of 1 nanometer in diameter. Immersion of a nanopore in a conducting fluid and application of a potential across it results in a slight electrical current due to conduction of ions through the nanopore. The amount of current which flows is sensitive to the size of the nanopore. As a DNA molecule passes through a nanopore, each nucleotide on the DNA molecule obstructs the nanopore to a different degree. Thus, the change in the current passing through the nanopore as the DNA molecule passes through the nanopore represents a reading of the DNA sequence.

Another example of a sequencing technique that can be used in the methods of the provided invention involves using a chemical-sensitive field effect transistor (chemFET) array to sequence DNA (for example, as described in US Patent Application Publication No. 20090026082). In one example of the technique, DNA molecules can be placed into reaction chambers, and the template molecules can be hybridized to a sequencing primer bound to a polymerase. Incorporation of one or more triphosphates into a new nucleic acid strand at the 3' end of the sequencing primer can be detected by a change in current by a chemFET. An array can have multiple chemFET sensors. In another example, single nucleic acids can be attached to beads, and the nucleic acids can be amplified on the bead, and the individual beads can be transferred to individual reaction chambers on a chemFET array, with each chamber having a chemFET sensor, and the nucleic acids can be sequenced.

Another example of a sequencing technique that can be is an electron microscope (Moudrianakis E. N. and Beer M. Proc Natl Acad Sci USA. 1965 March; 53:564-71). In one example of the technique, individual DNA molecules are labeled using metallic labels that are distinguishable using an electron microscope. These molecules are then stretched on a flat surface and imaged using an electron microscope to measure sequences.

In a particular embodiment, the sequencing is single-molecule sequencing-by-synthesis. Single-molecule sequencing is shown for example in Lapidus et al. (U.S. patent number 7,169,560), Quake et al. (U.S. patent number 6,818,395), Harris (U.S. patent number 7,282,337), Quake et al. (U.S. patent application number 2002/0164629), and Braslavsky, et al., PNAS (USA), 100: 3960-3964 (2003).

Briefly, a single-stranded nucleic acid (e.g., DNA or cDNA) is hybridized to oligonucleotides attached to a surface of a flow cell. The single-stranded nucleic acids may be captured by methods known in the art, such as those shown in Lapidus (U.S. patent number 7,666,593). The oligonucleotides may be covalently attached to the surface or various attachments other than covalent linking as known to those of ordinary skill in the art may be employed. Moreover, the attachment may be indirect, e.g., via the polymerases of the invention directly or indirectly attached to the surface. The surface may be planar or otherwise, and/or may be porous or non-porous, or any other type of surface known to those of ordinary skill to be suitable for attachment. The nucleic acid is then sequenced by imaging the polymerase-mediated addition of fluorescently-labeled nucleotides incorporated into the growing strand surface oligonucleotide, at single molecule resolution.

Methods wherein the nucleic acid sequencing reaction comprises hybridizing a sequencing primer to a single-stranded region of a linearized amplification product, sequentially incorporating one or more nucleotides into a polynucleotide strand complementary to the region of amplified template strand to be sequenced, identifying the base present in one or more of the incorporated nucleotide(s) and thereby determining the sequence of a region of the template strand are known.

For the sequence reconstruction process, short reads are stitched together bioinformatically by finding overlaps and extending them. To be able to do that unambiguously, one must ensure that long fragments that were amplified are distinct enough, and do not have similar stretches of DNA that will make assembly from short fragments ambiguous, which can occur, for example, if two molecules in a same well originated from overlapping positions on homologous chromosomes, overlapping positions of same chromosome, or genomic repeat. Such fragments can be detected during sequence assembly process by observing multiple possible ways to extend the fragment, one of which contains sequence specific to end marker. End markers can be chosen such that end marker sequence is not frequently found in DNA fragments of sample that is analyzed and probabilistic framework utilizing quality scores can be applied to decide whether a certain possible sequence extension way represents end maker and thus end of the fragment.

Overlapping fragments may be computationally discarded since they no longer represent the same initial long molecule. This process allows to treat population of molecules resulting after amplification as a clonally amplified population of disjoint molecules with no significant overlap or homology, which enables sequencing errors to be corrected to achieve very high consensus accuracy and allows unambiguous reconstruction of long fragments. If overlaps are not discarded, then one has to assume that reads may be originating from fragments originating from two homologous chromosomes or overlapping regions of the same chromosome (in case of diploid organism) which makes error correction difficult and ambiguous.

Computational removal of overlapping fragments obtained from both the 5' and the 3' directions also allows use of quality scores to resolve nearly-identical repeats. Resulting long fragments may be assembled into full genomes using any of the algorithms known in the art for genome sequence assembly that can utilize long reads.

In addition to de-novo assembly fragments can be used to obtain phasing (assignment to homologous copies of chromosomes) of genomic variants, by observing that under conditions of experiment described in the preferred embodiment long fragments originate from either one of chromosomes, which enables to correlate and co-localize variants detected in overlapping fragments obtained from distinct partitioned portions.

Amplification based detection

In certain embodiments, amplification based methods are used to detect the code site. Amplification refers to production of additional copies of a nucleic acid sequence and is generally carried out using polymerase chain reaction or other technologies well known in the art (e.g., Dieffenbach and Dveksler, PCR Primer, a Laboratory Manual, Cold Spring Harbor Press, Plainview, N.Y. [1995]). The amplification reaction may be any amplification reaction known in the art that amplifies nucleic acid molecules, such as polymerase chain reaction, nested polymerase chain reaction, polymerase chain reaction-single strand conformation polymorphism, ligase chain reaction (Barany F. (1991) PNAS 88:189-193; Barany F. (1991) PCR Methods and Applications 1:5-16), ligase detection reaction (Barany F. (1991) PNAS 88:189-193), strand displacement amplification and restriction fragments length polymorphism, transcription based amplification system, nucleic acid sequence-based amplification, rolling circle amplification, and hyper-branched rolling circle amplification.

Polymerase chain reaction (PCR) refers to methods by K. B. Mullis (U.S. patent numbers 4,683,195 and 4,683,202,) for increasing concentration of a segment of a target sequence in a mixture of genomic DNA without cloning or purification. The process for amplifying the target sequence includes introducing an excess of primers (oligonucleotides) to a DNA mixture containing a desired target sequence, followed by a precise sequence of thermal cycling. The present invention includes, but is not limited to, various PCR strategies as are known in the art, for example QPCR, multiplex PCR, assymetric PCR, nested PCR, hotstart PCR, touchdown PCR, assembly PCR, digital PCR, allele specific PCR, methylation specific PCR, reverse transcription PCR, helicase dependent PCR, inverse PCR, intersequence specific PCR, ligation mediated PCR, mini primer PCR, and solid phase PCR, emulsion PCR, and PCR as performed in a thermocycler, droplets, microfluidic reaction chambers, flow cells and other microfluidic devices.

In specific embodiments, digital PCR is used to detect the code sites. For digital PCR embodiments, after the first and second binders have been incubated with the pool of nucleic acids, the pool is diluted so that the sample can be compartmentalized in a manner in which each compartment includes on a single nucleic acid. Any type of compartment generally used for digital PCR may be used with methods of the invention. Exemplary compartments include chambers, wells, droplets, reaction volumes, slugs.

Poisson statistics dictate the dilution requirements needed to insure that each compartment contains only a single nucleic acid. In particular, the sample concentration should be dilute enough that most of the compartments contain no more than a single nucleic acid with only a small statistical chance that a compartment will contain two or more molecules. The parameters which govern this relationship are the volume of the compartment and the concentration of nucleic acid in the sample solution. The probability that a compartment will contain two or more nucleic acid ($NAT_{\leq 2}$) can be expressed as:

$$NAT_{\leq 2} = 1 - \{1 + [NAT] \times V\} \times e^{-(NAT) \times V}$$

where "[NAT]" is the concentration of nucleic acid in units of number of molecules per cubic micron ($\mu m^3$), and $V$ is the volume of the compartment in units of $\mu m^3$. It will be appreciated that $NAT_{\leq 2}$ can be minimized by decreasing the concentration of nucleic acid in the sample solution.

In particular embodiments, the compartmentalized portions are droplets and compartmentalizing involves forming the droplets. Sample droplets may be formed by any method known in the art. The droplets are aqueous droplets that are surrounded by an immiscible carrier fluid. Methods of forming droplets are shown for example in Link et al. (U.S. patent application numbers 2008/0014589, 2008/0003142, and 2010/0137163), Stone et al. (U.S. patent number 7,708,949 and U.S. patent application number 2010/0172803), Anderson et al. (U.S. patent number 7,041,481 and which reissued as RE41,780) and European publication number EP2047910 to Raindance Technologies Inc.

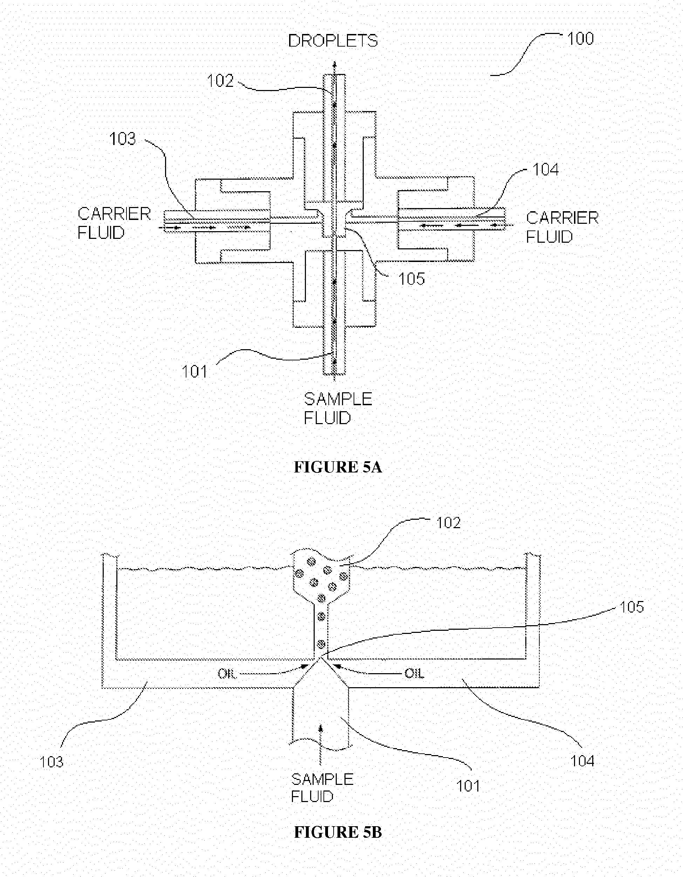

Figures 5A-B show an exemplary embodiment of a device **100** for droplet formation. Device **100** includes an inlet channel **101,** and outlet channel **102,** and two carrier fluid channels **103** and **104.** Channels **101, 102, 103,** and **104** meet at a junction **105.** Inlet channel **101** flows sample fluid to the junction **105.** Carrier fluid channels **103** and **104** flow a carrier fluid that is immiscible with the sample fluid to the junction **105.** Inlet channel **101** narrows at its distal portion wherein it connects to junction **105** (See Figure 5B). Inlet channel **101** is oriented to be perpendicular to carrier fluid channels **103** and **104.** Droplets are formed as sample fluid flows from inlet channel **101** to junction **105,** where the sample fluid interacts with flowing carrier fluid provided to the junction **105** by carrier fluid channels **103** and **104.** Outlet channel **102** receives the droplets of sample fluid surrounded by carrier fluid.

The sample fluid is typically an aqueous buffer solution, such as ultrapure water (e.g., 18 mega-ohm resistivity, obtained, for example by column chromatography), 10 mM Tris HCl and 1 mM EDTA (TE) buffer, phosphate buffer saline (PBS) or acetate buffer. Any liquid or buffer that is physiologically compatible with nucleic acid molecules can be used. The carrier fluid is one that is immiscible with the sample fluid. The carrier fluid can be a non-polar solvent, decane (e g., tetradecane or hexadecane), fluorocarbon oil, silicone oil or another oil (for example, mineral oil).

In certain embodiments, the carrier fluid contains one or more additives, such as agents which reduce surface tensions (surfactants). Surfactants can include Tween, Span, fluorosurfactants, and other agents that are soluble in oil relative to water. In some applications, performance is improved by adding a second surfactant to the sample fluid. Surfactants can aid in controlling or optimizing droplet size, flow and uniformity, for example by reducing the shear force needed to extrude or inject droplets into an intersecting channel. This can affect droplet volume and periodicity, or the rate or frequency at which droplets break off into an intersecting channel. Furthermore, the surfactant can serve to stabilize aqueous emulsions in fluorinated oils from coalescing.

In certain embodiments, the droplets may be coated with a surfactant. Preferred surfactants that may be added to the carrier fluid include, but are not limited to, surfactants such as sorbitan-based carboxylic acid esters (e.g., the "Span" surfactants, Fluka Chemika), including sorbitan monolaurate (Span 20), sorbitan monopalmitate (Span 40), sorbitan monostearate (Span 60) and sorbitan monooleate (Span 80), and perfluorinated polyethers (e.g., DuPont Krytox 157 FSL, FSM, and/or FSH). Other non-limiting examples of non-ionic surfactants which may be used include polyoxyethylenated alkylphenols (for example, nonyl-, p-dodecyl-, and dinonylphenols), polyoxyethylenated straight chain alcohols, polyoxyethylenated polyoxypropylene glycols, polyoxyethylenated mercaptans, long chain carboxylic acid esters (for example, glyceryl and polyglyceryl esters of natural fatty acids, propylene glycol, sorbitol, polyoxyethylenated sorbitol esters, polyoxyethylene glycol esters, etc.) and alkanolamines (e.g., diethanolamine-fatty acid condensates and isopropanolamine-fatty acid condensates).

In certain embodiments, the carrier fluid may be caused to flow through the outlet channel so that the surfactant in the carrier fluid coats the channel walls. In one embodiment, the fluorosurfactant can be prepared by reacting the perflourinated polyether DuPont Krytox 157 FSL, FSM, or FSH with aqueous ammonium hydroxide in a volatile fluorinated solvent. The solvent and residual water and ammonia can be removed with a rotary evaporator. The surfactant can then be dissolved (e.g., 2.5 wt %) in a fluorinated oil (e.g., Flourinert (3M)), which then serves as the carrier fluid.

Methods for performing PCR in droplets are shown for example in Link et al. (U.S. patent application numbers 2008/0014589, 2008/0003142, and 2010/0137163), Anderson et al. (U.S. patent number 7,041,481 and which reissued as RE41,780) and European publication number EP2047910 to Raindance Technologies Inc.

The sample droplet may be pre-mixed with a primer or primers, or the primer or primers may be added to the droplet. Aong with the primers, reagents for a PCR reaction are also introduced to the droplets. Such reagents generally include Taq polymerase, deoxynucleotides of type A, C, G and T, magnesium chloride, all suspended within an aqueous buffer. The droplet also includes detectably labeled probes for detection of the amplified target nucleic acid, the details of which are discussed below.

An exemplary method of introducing primers, PCR reagents, and probes to a sample droplet is as follows. After formation of the sample droplet from the first sample fluid, the droplet is contacted with a flow of a second sample fluid stream, which contains the primers for both the first and second binders. Contact between the droplet and the fluid stream results in a portion of the fluid stream integrating with the droplet to form a mixed droplet containing a nucleic having bound binders, primers, PCR reagents, and probes.

Droplets of the first sample fluid flow through a first channel separated from each other by immiscible carrier fluid and suspended in the immiscible carrier fluid. The droplets are delivered to the merge area, i.e., junction of the first channel with the second channel, by a pressure-driven flow generated by a positive displacement pump. While droplet arrives at the merge area, a bolus of a second sample fluid is protruding from an opening of the second channel into the first channel. The intersection of the channels may be perpendicular. However, any angle that results in an intersection of the channels may be used, and methods of the invention are not limited to the orientation of the channels.

The bolus of the second sample fluid stream continues to increase in size due to pumping action of a positive displacement pump connected to the second channel, which outputs a steady stream of the second sample fluid into the merge area. The flowing droplet containing the first sample fluid eventually contacts the bolus of the second sample fluid that is protruding into the first channel. Contact between the two sample fluids results in a portion of the second sample fluid being segmented from the second sample fluid stream and joining with the first sample fluid droplet 201 to form a mixed droplet.

In order to achieve the merge of the first and second sample fluids, the interface separating the fluids must be ruptured. In certain embodiments, this rupture can be achieved through the application of an electric charge. In certain embodiments, the rupture will result from application of an electric field. In certain embodiments, the rupture will be achieved through non-electrical means, e.g. by hydrophobic/hydrophilic patterning of the surface contacting the fluids.

Description of applying electric charge to sample fluids is provided in Link et al. (U.S. patent application number 2007/0003442) and European Patent Number EP2004316 to Raindance Technologies Inc.

Electric charge may be created in the first and second sample fluids within the carrier fluid using any suitable technique, for example, by placing the first and second sample fluids within an electric field (which may be AC, DC, etc.), and/or causing a reaction to occur that causes the first and second sample fluids to have an electric charge, for example, a chemical reaction, an ionic reaction, a photocatalyzed reaction, etc.

The electric field, in some embodiments, is generated from an electric field generator, i.e., a device or system able to create an electric field that can be applied to the fluid. The electric field generator may produce an AC field (i.e., one that varies periodically with respect to time, for example, sinusoidally, sawtooth, square, etc.), a DC field (i.e., one that is constant with respect to time), a pulsed field, etc. The electric field generator may be constructed and arranged to create an electric field within a fluid contained within a channel or a microfluidic channel. The electric field generator may be integral to or separate from the fluidic system containing the channel or microfluidic channel, according to some embodiments.

Techniques for producing a suitable electric field (which may be AC, DC, etc.) are known to those of ordinary skill in the art. For example, in one embodiment, an electric field is produced by applying voltage across a pair of electrodes, which may be positioned on or embedded within the fluidic system (for example, within a substrate defining the channel or microfluidic channel), and/or positioned proximate the fluid such that at least a portion of the electric field interacts with the fluid. The electrodes can be fashioned from any suitable electrode material or materials known to those of ordinary skill in the art, including, but not limited to, silver, gold, copper, carbon, platinum, tungsten, tin, cadmium, nickel, indium tin oxide ("ITO"), etc., as well as combinations thereof. In some cases, transparent or substantially transparent electrodes can be used.

The electric field facilitates rupture of the interface separating the second sample fluid and the droplet. Rupturing the interface facilitates merging of the bolus of the second sample fluid and the first sample fluid droplet. The forming mixed droplet continues to increase in size until it a portion of the second sample fluid breaks free or segments from the second sample fluid stream prior to arrival and merging of the next droplet containing the first sample fluid. The segmenting of the portion of the second sample fluid from the second sample fluid stream occurs as soon as the force due to the shear and/or elongational flow that is exerted on the forming mixed droplet by the immiscible carrier fluid overcomes the surface tension whose action is to keep the segmenting portion of the second sample fluid connected with the second sample fluid stream. The now fully formed mixed droplet continues to flow through the first channel.

Primers can be prepared by a variety of methods including but not limited to cloning of appropriate sequences and direct chemical synthesis using methods well known in the art (Narang et al., Methods Enzymol., 68:90 (1979); Brown et al., Methods Enzymol., 68:109 (1979)). Primers can also be obtained from commercial sources such as Operon Technologies, Amersham Pharmacia Biotech, Sigma, and Life Technologies. The primers can have an identical melting temperature. The lengths of the primers can be extended or shortened at the 5' end or the 3' end to produce primers with desired melting temperatures. Also, the annealing position of each primer pair can be designed such that the sequence and, length of the primer pairs yield the desired melting temperature. The simplest equation for determining the melting temperature of primers smaller than 25 base pairs is the Wallace Rule (Td=2(A+T)+4(G+C)). Computer programs can also be used to design primers, including but not limited to Array Designer Software (Arrayit Inc.), Oligonucleotide Probe Sequence Design Software for Genetic Analysis (Olympus Optical Co.), NetPrimer, and DNAsis from Hitachi Software Engineering. The TM (melting or annealing temperature) of each primer is calculated using software programs such as Oligo Design, available from Invitrogen Corp.

Once final droplets have been produced, the droplets are thermal cycled, resulting in amplification of the target nucleic acid in each droplet. The droplets are then heated to a temperature sufficient for dissociating the binders from the nucleic acids (e.g., 94o-100o Celsius). The droplets are maintained at that temperature for a sufficient time to allow dissociation (e.g., 2-5 minutes). The droplets are then cooled to a temperature sufficient for allowing one or more of the PCR reagents (e.g., primers) to anneal/hybridize to the binders (e.g., 50o-65o Celsius). This temperature is maintained r a sufficient time to allow annealing (e.g., 20-45 seconds). The droplets are then heated to a temperature sufficient for allowing extension of the primer (e.g., 68o-72o Celsius). The temperature is maintained for a sufficient time to allow extension of the primer (~ 1 min/kb). These cycles of denaturing, annealing and extension can be repeated for 20-45 additional cycles, resulting in amplification of the binder in each droplet.

During amplification, fluorescent signal is generated in a TaqMan assay by the enzymatic degradation of the fluorescently labeled probe. The probe contains a dye and quencher that are maintained in close proximity to one another by being attached to the same probe. When in close proximity, the dye is quenched by fluorescence resonance energy transfer to the quencher. Certain probes are designed that hybridize to the first binders, and other probes are designed that hybridize to the second binders. Probes that hybridize to the first binders have a different fluorophore attached than probes that hybridize to the second binders.

During the PCR amplification, the amplicon is denatured allowing the probe and PCR primers to hybridize. The PCR primer is extended by Taq polymerase replicating the alternative strand. During the replication process the Taq polymerase encounters the probe which is also hybridized to the same strand and degrades it. This releases the dye and quencher from the probe which are then allowed to move away from each other. This eliminates the FRET between the two, allowing the dye to release its fluorescence. Through each cycle more fluorescence is released. The amount of fluorescence released depends on the efficiency of the PCR reaction and also the kinetics of the probe hybridization. If there is a single mismatch between the probe and the target sequence the probe will not hybridize as efficiently and thus a fewer number of probes are degraded during each round of PCR and thus less fluorescent signal is generated. This difference in fluorescence per droplet can be detected and counted. The efficiency of hybridization can be affected by such things as probe concentration, probe ratios between competing probes, and the number of mismatches present in the probe.

Analysis

Analysis is then performed on the binders. Regardless of the code detection method (e.g., sequencing or amplification based methods), the analysis may be based on counting, i.e., determining a number of droplets or barcodes for the first binder, determining a number of droplets or barcode for the second binds, and then determining whether a statistical different exists between the first and second numbers. Such methods are well known in the art. See, e.g., Lapidus et al. (U.S. patent numbers 5,670,325 and 5,928,870) and Shuber et al. (U.S. patent number 6,203,993 and 6,214,558).

Fetal Aneuploidy

Fetal aneuploidy (e.g., Down syndrome, Edward syndrome, and Patau syndrome) and other chromosomal aberrations affect 9 of 1,000 live births (Cunningham et al. in Williams Obstetrics, McGraw-Hill, New York, p. 942, 2002). Chromosomal abnormalities are generally diagnosed by karyotyping of fetal cells obtained by invasive procedures such as chorionic villus sampling or amniocentesis. Those procedures are associated with potentially significant risks to both the fetus and the mother. Noninvasive screening using maternal serum markers or ultrasound are available but have limited reliability (Fan et al., PNAS, 105(42):16266-16271, 2008).

Methods of the invention may be used to screen for fetal aneuploidy. Such methods involve obtaining a sample, e.g., a tissue or body fluid, that is suspected to include both maternal and fetal nucleic acids. Such samples may include saliva, urine, tear, vaginal secretion, amniotic fluid, breast fluid, breast milk, sweat, or tissue. In certain embodiments, this sample is drawn maternal blood, and circulating DNA is found in the blood plasma, rather than in cells. A preferred sample is maternal peripheral venous blood.

In certain embodiments, approximately 10-20 mL of blood is drawn. That amount of blood allows one to obtain at least about 10,000 genome equivalents of total nucleic acid (sample size based on an estimate of fetal nucleic acid being present at roughly 25 genome equivalents/mL of maternal plasma in early pregnancy, and a fetal nucleic acid concentration of about 3.4% of total plasma nucleic acid). However, less blood may be drawn for a genetic screen where less statistical significance is required, or the nucleic acid sample is enriched for fetal nucleic acid.

Because the amount of fetal nucleic acid in a maternal sample generally increases as a pregnancy progresses, less sample may be required as the pregnancy progresses in order to obtain the same or similar amount of fetal nucleic acid from a sample.

In certain embodiments, the aneuploidy is trisomy of chromosome 21 (Down syndrome). However, the exemplified method herein can be sued for any fetal aneuploidy screen. In such embodiments, the target nucleic acid is nucleic acid of chromosome 21 and the first set of binders binds to the nucleic acid of chromosome 21 in the pool, and the second set of binders binds nucleic acid of a reference chromosome in the pool, such as chromosome 1. This is exemplified in Figure 6.

The methods are then conducted as described above and either sequencing or digital PCR can be used to detect the code site of the first and second binders. The detected code sites are then counted. Under the assumptions that first binders N and second binders M are in equal number and the Target region and Normalization region are in a fixed ratio to each other in the sample, i.e. 1:1 (nomal DNA) or 1:1.5 (trisomy 21) or 1:1.05 (blend of DNA from 90% normal cells and 10% cells with trisomy 21), then the number of first binders and second binders that bind to the sample DNA will be in the same ratio. A ratio that is not 1:1, e.g. 1:1.5 indicates trisomy 21 of the fetus.

Cancer

Methods may be used to generally screen for cancer. In this embodiment, the first set of binders binds genomic regions of the nucleic acids associated with known mutations involved in different cancers and the second set of binders binds genomic regions of the nucleic acids that are not mutated.

The methods are then conducted as described above and either sequencing or digital PCR can be used to detect the code site of the first and second binders. The detected code sites are then counted. How counting based methods can be used to screen for a cancer are known in the art. See, e.g., Lapidus et al. (U.S. patent numbers 5,670,325 and 5,928,870) and Shuber et al. (U.S. patent number 6,203,993 and 6,214,558).

Mutations that are indicative of cancer are known in the art. See for example, Hesketh (The Oncogene Facts Book, Academic Press Limited, 1995). Biomarkers associated with development of breast cancer are shown in Erlander et al. (US 7,504,214), Dai et al. (US 7,514, 209 and 7,171,311), Baker et al. (US 7,056,674 and US 7,081,340), Erlander et al. (US 2009/0092973). Biomarkers associated with development of cervical cancer are shown in Patel (US 7,300,765), Pardee et al. (US 7,153,700), Kim (US 6,905,844), Roberts et al. (US 6,316,208), Schlegel (US 2008/0113340), Kwok et al. (US 2008/0044828), Fisher et al. (US 2005/0260566), Sastry et al. (US 2005/0048467), Lai (US 2008/0311570) and Van Der Zee et al. (US 2009/0023137). Biomarkers associated with development of vaginal cancer are shown in Giordano (US 5,840,506), Kruk (US 2008/0009005), Hellman et al. (Br J Cancer. 100(8):1303-1314, 2009). Biomarkers associated with development of brain cancers (e.g., glioma, cerebellum, medulloblastoma, astrocytoma, ependymoma, glioblastoma) are shown in D'Andrea (US 2009/0081237), Murphy et al. (US 2006/0269558), Gibson et al. (US 2006/0281089), and Zetter et al. (US 2006/0160762). Biomarkers associated with development of renal cancer are shown in Patel (US 7,300,765), Soyupak et al. (US 7,482,129), Sahin et al. (US 7,527,933), Price et al. (US 7,229,770), Raitano (US 7,507,541), and Becker et al. (US 2007/0292869). Biomarkers associated with development of hepatic cancers (e.g., hepatocellular carcinoma) are shown in Horne et al. (US 6,974,667), Yuan et al. (US 6,897,018), Hanausek-Walaszek et al. (US 5,310,653), and Liew et al. (US 2005/0152908). Biomarkers associated with development of gastric, gastrointestinal, and/or esophageal cancers are shown in Chang et al. (US 7,507,532), Bae et al. (US 7,368,255), Muramatsu et al. (US 7,090,983), Sahin et al. (US 7,527,933), Chow et al. (US 2008/0138806), Waldman et al. (US 2005/0100895), Goldenring (US 2008/0057514), An et al. (US 2007/0259368), Guilford et al. (US 2007/0184439), Wirtz et al. (US 2004/0018525), Filella et al. (Acta Oncol. 33(7):747-751, 1994), Waldman et al. (US 6,767,704), and Lipkin et al. (Cancer Research, 48:235-245, 1988). Biomarkers associated with development of ovarian cancer are shown in Podust et al. (US 7,510,842), Wang (US 7,348,142), O'Brien et al. (US 7,291,462, 6,942,978, 6,316,213, 6,294,344, and 6,268,165), Ganetta (US 7,078,180), Malinowski et al. (US 2009/0087849), Beyer et al. (US 2009/0081685), Fischer et al. (US 2009/0075307), Mansfield et al. (US 2009/0004687), Livingston et al. (US 2008/0286199), Farias-Eisner et al. (US 2008/0038754), Ahmed et al. (US 2007/0053896), Giordano (US 5,840,506), and Tchagang et al. (Mol Cancer Ther, 7:27-37, 2008). Biomarkers associated with development of head-and-neck and thyroid cancers are shown in Sidransky et al. (US 7,378,233), Skolnick et al. (US 5,989,815), Budiman et al. (US 2009/0075265), Hasina et al. (Cancer Research, 63:555-559, 2003), Kebebew et al. (US 2008/0280302), and Ralhan (Mol Cell Proteomics, 7(6):1162-1173, 2008). Biomarkers associated with development of colorectal cancers are shown in Raitano et al. (US 7,507,541), Reinhard et al. (US 7,501,244), Waldman et al. (US 7,479,376); Schleyer et al. (US 7,198,899); Reed (US 7,163,801), Robbins et al. (US 7,022,472), Mack et al. (US 6,682,890), Tabiti et al. (US 5,888,746), Budiman et al. (US 2009/0098542), Karl (US 2009/0075311), Arjol et al. (US 2008/0286801), Lee et al. (US 2008/0206756), Mori et al. (US 2008/0081333), Wang et al. (US 2008/0058432), Belacel et al. (US 2008/0050723), Stedronsky et al. (US 2008/0020940), An et al. (US 2006/0234254), Eveleigh et al. (US 2004/0146921), and Yeatman et al. (US 2006/0195269). Biomarkers associated with development of prostate cancer are shown in Sidransky (US 7,524,633), Platica (US 7,510,707), Salceda et al. (US 7,432,064 and US 7,364,862), Siegler et al. (US 7,361,474), Wang (US 7,348,142), Ali et al. (US 7,326,529), Price et al. (US 7,229,770), O'Brien et al. (US 7,291,462), Golub et al. (US 6,949,342), Ogden et al. (US 6,841,350), An et al. (US 6,171,796), Bergan et al. (US 2009/0124569), Bhowmick (US 2009/0017463), Srivastava et al. (US 2008/0269157), Chinnaiyan et al. (US 2008/0222741), Thaxton et al. (US 2008/0181850), Dahary et al. (US 2008/0014590), Diamandis et al. (US 2006/0269971), Rubin et al. (US 2006/0234259), Einstein et al. (US 2006/0115821), Paris et al. (US 2006/0110759), Condon-Cardo (US 2004/0053247), and Ritchie et al. (US 2009/0127454). Biomarkers associated with development of pancreatic cancer are shown in Sahin et al. (US 7,527,933), Rataino et al. (US 7,507,541), Schleyer et al. (US 7,476,506), Domon et al. (US 7,473,531), McCaffey et al. (US 7,358,231), Price et al. (US 7,229,770), Chan et al. (US 2005/0095611), Mitchl et al. (US 2006/0258841), and Faca et al. (PLoS Med 5(6):e123, 2008). Biomarkers associated with development of lung cancer are shown in Sahin et al. (US 7,527,933), Hutteman (US 7,473,530), Bae et al. (US 7,368,255), Wang (US 7,348,142), Nacht et al. (US 7,332,590), Gure et al. (US 7,314,721), Patel (US 7,300,765), Price et al. (US 7,229,770), O'Brien et al. (US 7,291,462 and US 6,316,213), Muramatsu et al. (US 7,090,983), Carson et al. (US 6,576,420), Giordano (US 5,840,506), Guo (US 2009/0062144), Tsao et al. (US 2008/0176236), Nakamura et al. (US 2008/0050378), Raponi et al. (US 2006/0252057), Yip et al. (US 2006/0223127), Pollock et al. (US 2006/0046257), Moon et al. (US 2003/0224509), and Budiman et al. (US 2009/0098543). Biomarkers associated with development of skin cancer (e.g., basal cell carcinoma, squamous cell carcinoma, and melanoma) are shown in Roberts et al. (US 6,316,208), Polsky (US 7,442,507), Price et al. (US 7,229,770), Genetta (US 7,078,180), Carson et al. (US 6,576,420), Moses et al. (US 2008/0286811), Moses et al. (US 2008/0268473), Dooley et al. (US 2003/0232356), Chang et al. (US 2008/0274908), Alani et al. (US 2008/0118462), Wang (US 2007/0154889), and Zetter et al. (US 2008/0064047). Biomarkers associated with development of multiple myeloma are shown in Coignet (US 7,449,303), Shaughnessy et al. (US 7,308,364), Seshi (US 7,049,072), and Shaughnessy et al. (US 2008/0293578, US 2008/0234139, and US 2008/0234138). Biomarkers associated with development of leukemia are shown in Ando et al. (US 7,479,371), Coignet (US 7,479,370 and US 7,449,303), Davi et al. (US 7,416,851), Chiorazzi (US 7,316,906), Seshi (US 7,049,072), Van Baren et al. (US 6,130,052), Taniguchi (US 5,643,729), Insel et al. (US 2009/0131353), and Van Bockstaele et al. (Blood Rev. 23(1):25-47, 2009). Biomarkers associated with development of lymphoma are shown in Ando et al. (US 7,479,371), Levy et al. (US 7,332,280), and Arnold (US 5,858,655). Biomarkers associated with development of bladder cancer are shown in Price et al. (US 7,229,770), Orntoft (US 6,936,417), Haak-Frendscho et al. (US 6,008,003), Feinstein et al. (US 6,998,232), Elting et al. (US 2008/0311604), and Wewer et al. (2009/0029372).

<u>Detection of chromosomal aneuploidy using probes that hybridize to multiple locations in different chromosomes at multiple sites coupled with sequence specific PCR primers</u>

Identification of an aneuploidy from DNA extracted from maternal blood using end point TAQMAN (hydrolysis probes, Invitrogen, Inc.) based digital PCR may be accomplished by conducting one assay against an affected chromosome and another assay against a normalization chromosome. The number of counts is then compared in order to identify a statistically relevant elevation of the number of counts. This is readily done in a multiplex fashion using a different color dye for each assay. Often there is either insufficient DNA or the fetal fraction of the DNA is insufficient to identify aneuploidies. One way of overcoming the limitation of insufficient material is to use multiple assays for each chromosome. That is a rather limited approach, if each assay were to use a different fluorescence wavelength. However, by using the same dye for assays on the same chromosome, chromosomal identity is maintained even though the exact target may not be identified. For example, 10 assays for each chromosome are tuned to have roughly the same end point fluorescence intensity in order to indicate the presence of one of 10 possible targets on a chromosome. Doing so increases the number of amplifiable targets in a sample by a factor of 10. Figure 7 provides a schematic diagram of this embodiment. In Figure 7, trisomy 21 is exemplified, however, this embodiment is not limited to trisomy 21 and may be used with other trisomies simultaneously or as separate assays.

Another approach is to use a set of probes that hybridize to multiple regions in the genome. Typically, great care is taken to verify that probes used in assays like TAQMAN (hydrolysis probes, Invitrogen, Inc.) will hybridize to only one region in the genome. However, an alternative strategy is to identify regions that are present at multiple sites throughout the genome. Those regions would ideally be widely distributed throughout the genome and be flanked by unique DNA sequences that would allow the design of sequence specific PCR primers that only amplify a single site in the genome which has a probe hybridized to it. The probe sequences could be spread throughout the entire genome or specific for only one chromosome. The greater the number of sites present in the genome and the more evenly spread out the probe sequence, the more universal the probe set will be. An advantage of this assay is that the probe concentration may be independent of the number of target sites.

Figure 8 provides a schematic of such an assay. In Figure 8, trisomy 21 is exemplified, however, this embodiment is not limited to trisomy 21 and may be used with other trisomies simultaneously or as separate assays. In this embodiment, to design a test for trisomy 21, probes and PCR primer combinations are selected for chromosome 21 and for a reference chromosome, such as chromosome 1. The particular probe that is selected for chromosome 21 is present in chromosome 21 at multiple locations. However, the probe site could also be present in other chromosomes, even chromosome 1. This probe would be labeled with dye-1. PCR primers are designed that flank the probe sites but that selectively amplify only one region in chromosome 21 that contains a probe for each PCR primer pair. The number of PCR primer pairs that are used could be a subset of the probe sites on chromosome 21 or the entire set on chromosome 21. Signal is generated at each site in which a probe is targeted that is also targeted by the PCR primers. Other regions to which the probe can hybridize do not generate signal unless also targeted by the PCR primers. This way even though the probes hybridize to other chromosomes or genomic regions they cannot generate signal. A second probe which also hybridizes to multiple locations in the genome and in this case at least to some regions on chromosome 1 is labeled with dye-2. Sequence specific PCR primers that flank some of the probes on chromosome 1 are designed. As with the first probe, it may hybridize to multiple other locations in the genome other than chromosome 1. However, only the probes targeted to chromosome 1 that also included in the amplicons generated by the PCR primers will produce signal. In this way a single probe with a single dye can target multiple regions in a particular chromosome and generate a signal in digital PCR. The ability to target multiple regions gives more statistical power to identify small changes in relative copy numbers between chromosomes that allow the identification of chromosomal aneuploidy. In most cases, the specific sequence of the probe is present on both chromosomes, thus making it impossible to identify if a specific target sequence is present in a reaction, only presence or absence of some portions of the chromosome can be determined, but not the target on the chromosome.

An alternative approach is to have multiple primers to each chromosome in which the primers have a chromosome specific probe location on the primer. Such an approach is shown schematically in Figure 9.

In the examples above, two different probes with two different dyes are designed to distinguish between signals generated for different chromosomes. Multiple chromosomes can also be targeted with a single dye by varying the signal produced by each probe. Because these reactions are carried out in droplets with very precise volumes, assay signal intensities can be adjusted by varying such things as the concentration of probes in each droplet. Two different probes with the same dye can be tuned to generate different final intensities in a droplet. In this way a droplet positive for one chromosome can be distinguished from another droplet containing a different chromosome.

In no way are these techniques restricted to use with droplets, and any form of partitioning the sample may be used.

## EXAMPLES

Example 1: Molecular Diagnostic Screening Assay

Hybridization reactions were set up containing 50 ng of genomic DNA, 3.75 femtomole each of two or four hybridization tiles (Table 1) with a 1x concentration of MLPA buffer (MRC-Holland) in a total volume of 25 μL.

Table 1. Hybridization Tile Sequences.

| Hybridization Tile | Sequence |
|---|---|
| DMDe8 Hyb A2 | 5-GATCGACGAGACACTCTCGCAGACCTGCTGGTGAGA GGCCAGCACCATGCAACTCCTT-3 (SEQ ID NO: 1) |
| DMDe8 Hyb B2 | 5-/5Phos/CAGCAACAGCAGCAGGAAGCAGTA TCATCGCCGGGAGATATACCCA-3 (SEQ ID NO: 2) |
| DMDi3 Hyb A2 | 5-GATCGACGAGACACTCTCGCAGACCTGCTGGTGAG ATTGTTTCTTCATTCTATAGCCCAGTTGG-3 (SEQ ID NO: 3) |
| DMDi3 Hyb B2 | 5-/5Phos/GGATTACTGCATGACCACCAATGACA GATCGCCGGGAGATATACCCA-3 (SEQ ID NO: 4) |

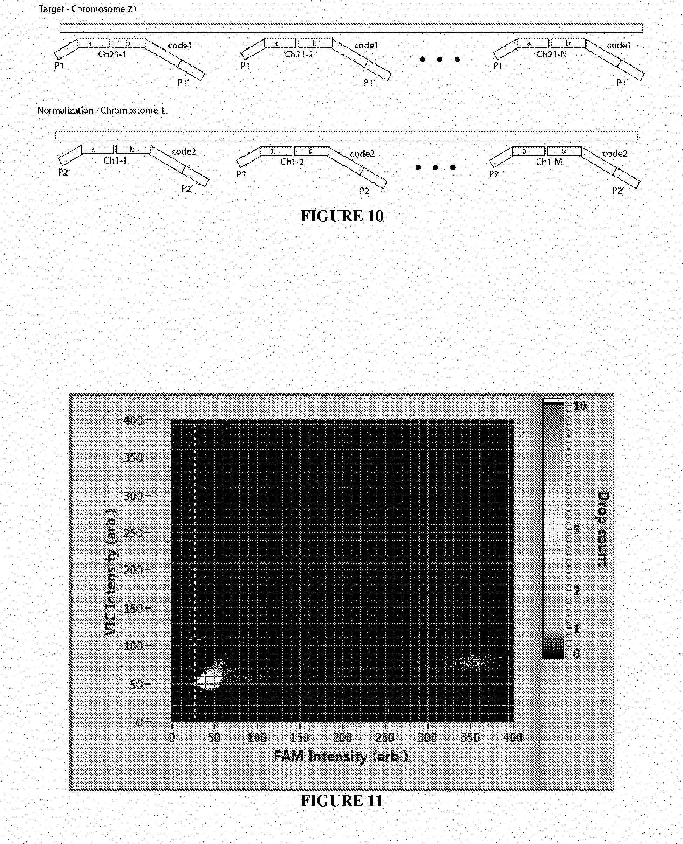

DMDe8 Hyb B2 and DMDi3 B2 are represented in FIG. 10 as the "P1 - a tile". DMDe8 Hyb A2 and DMDi3 A2 are represented in FIG. 10 as the "P1' - code 1 - b" tile. The DMDe8 Hyb A2 and DMDe8 Hyb B2 tile combination is represented by the Chr21-1 tile combination in FIG. 10 and the DMDi3 Hyb A2 and DMDi3 Hyb B2 tile combination is represented in FIG. 10 as the Chr21-2 tile combination. Each tile is made up of two parts. The first part (hyb A) consists of a sequence specific region that will hybridize to the denatured DNA. The 5' end of hyb A also contains the forward primer sequence and the probe sequence. The second part of the tile (hyb B) contains a sequence specific region and the sequence for the reverse primer.

The genomic DNA was denatured at 98°C degrees for five minutes. The DNA was then added to a mixture of MLPA Buffer and 3.75 femtomol of tiles. Three separate hybridization reactions were set up:

1. The DMDe8 Hyb A2 + DMDe8 Hyb B2 tile
2. DMDi3 Hyb A2 + DMDi3 Hyb B2 tile
3. DMDe8 Hyb A2 + DMDe8 Hyb B2 tile + DMDi3 Hyb A2 + DMDi3 Hyb B2 tile

The samples were heated to 95°C degrees for one minute followed by 60°C for sixteen hours.

Following the hybridization step a ligation reaction was set up. Three $\mu$L each of Buffer A (MRC-Holland) and Buffer B (MRC-Holland) were mixed with one $\mu$L of Ligase (MRC-Holland). This mixture was brought to a final volume of 32 uL with water and mixed with the hybridization reaction. The samples were then cycled using the following conditions: 54°C for 15 minutes, 98°C degrees for 5 minutes and then a hold at 20°C until ready to continue.

A PCR reaction mix was made consisting of 1X Genotyping master mix (Life Technologies 4371355 - TaqMan Genotyping Buffer), 0.9uM PCR primer, RDT stabilizer, 0.2uM FAM probe and 3ul ligated sample. The ligation reactions were processed by the RainDance Source instrument to generate approximately 5 million 5pL droplets that were thermal cycled using the following conditions:

| | Temp | | Time | |
|---|---|---|---|---|
| 1 | 95 | °C | 10 | min |
| 2 | 95 | °C | 15 | sec |
| 3 | 58 | °C | 15 | sec |
| 4 | 60 | °C | 45 | sec |
| 5 | go to step 2 and repeat 44 times | | | |
| 6 | 4 | °C | Long | |

The primer and probe sequences used in the PCR master mix are shown in Tables 2 and 3.

**Table 2.** Primer Sequences

| Oligo/Probe | Sequence |
|---|---|
| Forward | 5- GAT CGA CGA GAC ACT CTC G -3(SEQ ID NO: 5) |
| Reverse | 5-TGG GTA TAT CTC CCG GCG AT -3(SEQ ID NO: 6) |

**Table 3.** Probe Sequences

| Oligo/Probe | Sequence |
|---|---|
| FAM Probe | 5- /56-FAM/+CCT +G+CT +G+GT +GA/3IABkFQ/ -3(SEQ ID NO: 7) |
| | |

Following thermal cycling the droplets were reinjected into a RainDance Sense instrument to detected the number of droplets with a positive FAM signal. The results are shown in FIGS. 11-13. FIGS. 11-13 show the scatter plot results for each of the three reactions. The white box in FIGS. 11-13 contain the FAM fluorescent positive droplets which indicate the presence of a DNA fragment corresponding to the X chromosome which is the target chromosome in this case. FIGS. 11-13 contain the count of PCR positive droplets (FAM Drops). The number of FAM positive droplets for each reaction were:

DMDi3 - 239 - FIG. 11

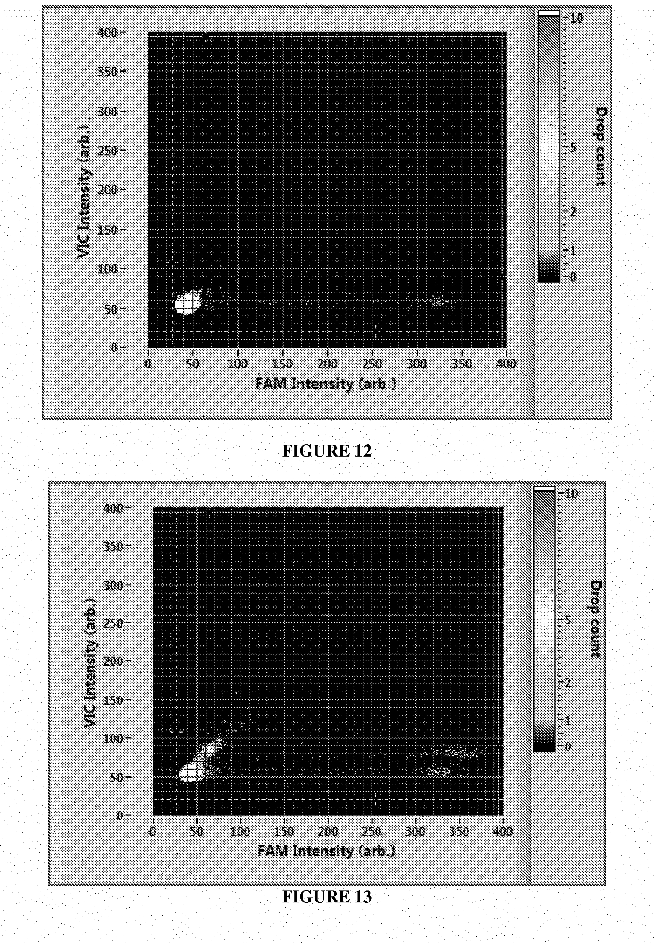

DMDe8 - 169 - FIG. 12
DMDi3 + DMDe8 - 398 - FIG. 13

The reaction with both sets of tiles (DMDi3 + DMDe8) resulted in approximately the same number of FAM positive droplets as the combination of the FAM positive droplets for the DMDi3 and DMDe8 reactions run separately. This demonstrates that the combination of multiple tile sets for different regions of the same chromosome results in the generation of more positive droplets in a single sample which results in a greater statistical power to identify chromosomal copy number differences in samples with a small percentage of fetal DNA.

**Claims**

1. A method for detecting aneuploidy, the method comprising:
   providing a first set of oligonucleotide binders and a second set of oligonucleotide binders, each of the oligonucleotide binders comprising:

   a 5' region comprising a first universal primer site;
   a 3' region comprising a second universal primer site; and
   a central region,
   wherein the central region of the oligonucleotide binders of the first set comprise target-specific sequences that bind to different regions on a target nucleic acid comprising chromosome 21 and a first code site, and the central region of the oligonucleotide binders of the second set comprise reference specific sequences that bind to different regions on a reference nucleic acid comprising a chromosome other than chromosome 21 and a second code site;
   exposing the sets of oligonucleotide binders to a sample pool comprising the target nucleic acid and the reference nucleic acid, wherein the first set binds to multiple regions on the target nucleic acid and the second set binds to multiple regions on the reference nucleic acid;
   removing the unbound oligonucleotide binders;
   diluting and compartmentalizing the sample in a plurality of droplets at a dilution sufficient that most of the droplets contain no more than a single target or reference nucleic acid bound to binders from the exposing step the droplets further comprising primer species that bind to the universal primer sites and first and second hydrolysis probes that bind the respective first and second code sites;
   amplifying the oligonucleotide binders in the droplets in the presence of the first and second probes labeled with a dye and a quencher, wherein the first probe releases a first fluorescent signal upon amplification, and the second probe releases a second fluorescent signal upon amplification, and wherein the first and second fluorescent signal are different;
   detecting the released first and second fluorescent signal in the droplets to thereby count numbers of the droplets comprising the first or second detectable label; and
   screening for aneuploidy by determining a statistical difference between the number of droplets comprising the first detectable label and the number of droplets comprising the second detectable label.

2. The method of claim 1, wherein the amplifying step comprises PCR.

3. The method of claim 2, wherein the PCR uses the primer species that bind to the universal primer sites.

4. The method of claim 1, wherein the first and second hydrolysis probes each comprise a Taqman probe.

5. The method of claim 1, wherein the first and second sets of oligonucleotide binders are hybridized in the same reaction.

6. The method of claim 1, wherein the first and second universal primer sites of the first set of oligonucleotide binders are different than the first and second universal primer sites of the second set of oligonucleotide binders.

7. The method of claim 1, wherein the code site of the first set of oligonucleotide binders is different than the code site of the second set of oligonucleotide binders.

8. The method of claim 1, wherein the droplets are aqueous droplets in an immiscible fluid.

**9.** The method of claim 8, wherein the immiscible fluid is an oil.

**10.** The method of claim 9, wherein the oil comprises a surfactant.

**Patentansprüche**

**1.** Verfahren zum Nachweisen von Aneuploidie, wobei das Verfahren Folgendes umfasst:
Bereitstellen eines ersten Satzes von Oligonukleotid-Bindern und eines zweiten Satzes von Oligonukleotid-Bindern, wobei jeder der Oligonukleotid-Binder Folgendes umfasst:

eine 5'-Region, die eine erste universelle Primer-Stelle umfasst;
eine 3'-Region, die eine zweite universelle Primer-Stelle umfasst; und
eine zentrale Region,
wobei die zentrale Region der Oligonukleotid-Binder des ersten Satzes zielspezifische Sequenzen umfasst, die an unterschiedliche Regionen auf einer Ziel-Nukleinsäure binden, die Chromosom 21 und eine erste Codestelle umfasst, und die zentrale Region der Oligonukleotid-Binder des zweiten Satzes referenzspezifische Sequenzen umfasst, die an unterschiedliche Regionen auf einer Referenz-Nukleinsäure binden, die ein anderes Chromosom als Chromosom 21 und eine zweite Codestelle umfasst;
Aussetzen der Sätze von Oligonukleotid-Bindern gegenüber einem Probenpool, der die Ziel-Nukleinsäure und die Referenz-Nukleinsäure umfasst, wobei der erste Satz an mehrere Regionen auf der Ziel-Nukleinsäure bindet und der zweite Satz an mehrere Regionen auf der Referenz-Nukleinsäure bindet;
Entfernen der ungebundenen Oligonukleotid-Binder;
Verdünnen und Kompartimentieren der Probe in eine Vielzahl von Tröpfchen in einer Verdünnung, die ausreicht, dass die meisten der Tröpfchen nicht mehr als eine einzige Ziel- oder Referenz-Nukleinsäure enthalten, die an Binder aus dem Expositionsschritt gebunden ist, wobei die Tröpfchen ferner Primer-Spezies, die an die universellen Primer-Stellen binden, und erste und zweite Hydrolyse-Sonden, die an die jeweiligen ersten und zweiten Codestellen binden, umfassen;
Amplifizieren der Oligonukleotid-Binder in den Tröpfchen in Anwesenheit der ersten und zweiten Sonden, die mit einem Farbstoff und einem Quencher markiert sind, wobei die erste Sonde bei der Amplifikation ein erstes Fluoreszenzsignal freisetzt und die zweite Sonde bei der Amplifikation ein zweites Fluoreszenzsignal freisetzt, und wobei das erste und zweite Fluoreszenzsignal unterschiedlich sind;
Nachweisen des freigesetzten ersten und zweiten Fluoreszenzsignals in den Tröpfchen, um dadurch die Anzahl der Tröpfchen zu zählen, die den ersten oder zweiten nachweisbaren Marker umfassen; und
Screening auf Aneuploidie durch Bestimmen einer statistischen Differenz zwischen der Anzahl der Tröpfchen, die den ersten nachweisbaren Marker umfassen, und der Anzahl der Tröpfchen, die den zweiten nachweisbaren Marker umfassen.

**2.** Verfahren nach Anspruch 1, wobei der Amplifikationsschritt PCR umfasst.

**3.** Verfahren nach Anspruch 2, wobei die PCR die Primer-Spezies verwendet, die an die universellen Primer-Stellen binden.

**4.** Verfahren nach Anspruch 1, wobei die erste und zweite Hydrolyse-Sonde jeweils eine Taqman-Sonde umfassen.

**5.** Verfahren nach Anspruch 1, wobei der erste und der zweite Satz von Oligonukleotid-Bindern in der gleichen Reaktion hybridisiert werden.

**6.** Verfahren nach Anspruch 1, wobei sich die ersten und zweiten universellen Primer-Stellen des ersten Satzes von Oligonukleotid-Bindern von den ersten und zweiten universellen Primer-Stellen des zweiten Satzes von Oligonukleotid-Bindern unterscheiden.

**7.** Verfahren nach Anspruch 1, wobei sich die Codestelle des ersten Satzes von Oligonukleotid-Bindern von der Codestelle des zweiten Satzes von Oligonukleotid-Bindern unterscheidet.

**8.** Verfahren nach Anspruch 1, wobei die Tröpfchen wässrige Tröpfchen in einem nicht mischbaren Fluid sind.

**9.** Verfahren nach Anspruch 8, wobei das nicht mischbare Fluid ein Öl ist.

**10.** Verfahren nach Anspruch 9, wobei das Öl ein Tensid umfasst.

**Revendications**

**1.** Procédé de détection d'aneuploïdie, le procédé comprenant :
la fourniture d'un premier ensemble de liants oligonucléotidiques et d'un second ensemble de liants oligonucléotidiques, chacun des liants oligonucléotidiques comprenant :

une région 5' comprenant un premier site d'amorce universelle ;
une région 3' comprenant un second site d'amorce universelle ; et
une région centrale,
dans lequel la région centrale des liants oligonucléotidiques du premier ensemble comprend des séquences spécifiques à une cible qui se lient à différentes régions sur un acide nucléique cible comprenant le chromosome 21 et un premier site de code, et la région centrale des liants oligonucléotidiques du second ensemble comprend des séquences spécifiques de référence qui se lient à différentes régions d'un acide nucléique de référence comprenant un chromosome autre que le chromosome 21 et un second site de code ;
l'exposition des ensembles de liants oligonucléotidiques à un regroupement d'échantillons comprenant l'acide nucléique cible et l'acide nucléique de référence, dans lequel le premier ensemble se lie à de multiples régions sur l'acide nucléique cible et le second ensemble se lie à de multiples régions sur l'acide nucléique de référence ;
l'élimination des liants oligonucléotidiques non liés ;
la dilution et la compartimentation de l'échantillon dans une pluralité de gouttelettes à une dilution suffisante pour que la plupart des gouttelettes ne contiennent pas plus d'un(e) seul(e) cible ou acide nucléique de référence lié aux liants issus de l'étape d'exposition, les gouttelettes comprenant en outre des espèces d'amorces qui se lient aux sites d'amorces universelles et des première et seconde sondes d'hydrolyse qui se lient aux premier et second sites de codes respectifs ;
l'amplification des liants oligonucléotidiques dans les gouttelettes en présence des première et seconde sondes marquées avec un colorant et un désactiveur, dans lequel la première sonde libère un premier signal fluorescent lors de l'amplification, et la seconde sonde libère un second signal fluorescent lors de l'amplification, et dans lequel les premier et second signaux fluorescents sont différents ;
la détection des premier et second signaux fluorescents libérés dans les gouttelettes pour ainsi compter le nombre des gouttelettes comprenant le premier ou second marqueur détectable ; et
le criblage de l'aneuploïdie par détermination d'une différence statistique entre le nombre de gouttelettes comprenant le premier marqueur détectable et le nombre de gouttelettes comprenant le second marqueur détectable.

**2.** Procédé selon la revendication 1, dans lequel l'étape d'amplification comprend la PCR.

**3.** Procédé selon la revendication 2, dans lequel la PCR utilise les espèces d'amorces qui se lient aux sites d'amorces universelles.

**4.** Procédé selon la revendication 1, dans lequel les première et seconde sondes d'hydrolyse comprennent chacune une sonde Taqman.

**5.** Procédé selon la revendication 1, dans lequel les premier et second ensembles de liants oligonucléotidiques sont hybridés dans la même réaction.

**6.** Procédé selon la revendication 1, dans lequel les premier et second sites d'amorces universelles du premier ensemble de liants oligonucléotidiques sont différents des premier et second sites d'amorces universelles du second ensemble de liants oligonucléotidiques.

**7.** Procédé selon la revendication 1, dans lequel le site de code du premier ensemble de liants oligonucléotidiques est différent du site de code du second ensemble de liants oligonucléotidiques.

**8.** Procédé selon la revendication 1, dans lequel les gouttelettes sont des gouttelettes aqueuses dans un fluide non miscible.

**9.** Procédé selon la revendication 8, dans lequel le fluide non miscible est une huile.

**10.** Procédé selon la revendication 9, dans lequel l'huile comprend un tensioactif.

**FIGURE 1**

**FIGURE 2**

**FIGURE 3**

**FIGURE 4**

**FIGURE 5A**

**FIGURE 5B**

**FIGURE 6**

**FIGURE 7**

**FIGURE 8**

**FIGURE 9**

Target - Chromosome 21

**FIGURE 10**

**FIGURE 11**

**FIGURE 12**

**FIGURE 13**

## REFERENCES CITED IN THE DESCRIPTION

**Patent documents cited in the description**

- US 7968287 B, Griffiths
- US 20080014589 A, Link
- WO 9928507 A1
- US 2011159499 A1
- EP 18259964 A
- WO 2006042303 A
- US 20020190663 A1
- US 5888778 A, Shuber
- US 7169560 B, Lapidus
- US 20090191565 A, Lapidus
- US 6818395 B, Quake
- US 7282337 B, Harris
- US 20020164629 A, Quake
- US 20090026082 A
- US 20090127589 A
- US 20100035252 A
- US 20100137143 A
- US 20100188073 A
- US 20100197507 A
- US 20100282617 A
- US 20100300559 A
- US 20100300895 A
- US 20100301398 A
- US 20100304982 A
- US 20090026082
- US 7666593 B, Lapidus
- US 4683195 A, K. B. Mullis
- US 4683202 A
- US 20080003142 A
- US 20100137163 A
- US 7708949 B, Stone
- US 20100172803 A
- US 7041481 B, Anderson
- EP 2047910 A
- US 20070003442 A, Link
- EP 2004316 A
- US 5670325 A, Lapidus
- US 5928870 A
- US 6203993 B, Shuber
- US 6214558 B
- US 7504214 B, Erlander
- US 7514 A, Dai
- US 209 A
- US 7171311 B
- US 7056674 B, Baker
- US 7081340 B
- US 20090092973 A, Erlander
- US 7300765 B, Patel
- US 7153700 B, Pardee

- US 6905844 B, Kim
- US 6316208 B, Roberts
- US 20080113340 A, Schlegel
- US 20080044828 A, Kwok
- US 20050260566 A, Fisher
- US 20050048467 A, Sastry
- US 20080311570 A, Lai
- US 20090023137 A, Van Der Zee
- US 5840506 A, Giordano
- US 20080009005 A, Kruk
- US 20090081237 A, D'Andrea
- US 20060269558 A, Murphy
- US 20060281089 A, Gibson
- US 20060160762 A, Zetter
- US 7482129 B, Soyupak
- US 7527933 B, Sahin
- US 7229770 B, Price
- US 7507541 B, Raitano
- US 20070292869 A, Becker
- US 6974667 B, Horne
- US 6897018 B, Yuan
- US 5310653 A, Hanausek-Walaszek
- US 20050152908 A, Liew
- US 7507532 B, Chang
- US 7368255 B, Bae
- US 7090983 B, Muramatsu
- US 20080138806 A, Chow
- US 20050100895 A, Waldman
- US 20080057514 A, Goldenring
- US 20070259368 A, An
- US 20070184439 A, Guilford
- US 20040018525 A, Wirtz
- US 6767704 B, Waldman
- US 7510842 B, Podust
- US 7348142 B, Wang
- US 7291462 B, O'Brien
- US 6942978 B
- US 6316213 B
- US 6294344 B
- US 6268165 B
- US 7078180 B, Ganetta
- US 20090087849 A, Malinowski
- US 20090081685 A, Beyer
- US 20090075307 A, Fischer
- US 20090004687 A, Mansfield
- US 20080286199 A, Livingston
- US 20080038754 A, Farias-Eisner
- US 20070053896 A, Ahmed
- US 7378233 B, Sidransky

- US 5989815 A, Skolnick
- US 20090075265 A, Budiman
- US 20080280302 A, Kebebew
- US 7501244 B, Reinhard
- US 7479376 B, Waldman
- US 7198899 B, Schleyer
- US 7163801 B, Reed
- US 7022472 B, Robbins
- US 6682890 B, Mack
- US 5888746 A, Tabiti
- US 20090098542 A, Budiman
- US 20090075311 A, Karl
- US 20080286801 A, Arjol
- US 20080206756 A, Lee
- US 20080081333 A, Mori
- US 20080058432 A, Wang
- US 20080050723 A, Belacel
- US 20080020940 A, Stedronsky
- US 20060234254 A, An
- US 20040146921 A, Eveleigh
- US 20060195269 A, Yeatman
- US 7524633 B, Sidransky
- US 7510707 B, Platica
- US 7432064 B, Salceda
- US 7364862 B
- US 7361474 B, Siegler
- US 7326529 B, Ali
- US 6949342 B, Golub
- US 6841350 B, Ogden
- US 6171796 B, An
- US 20090124569 A, Bergan
- US 20090017463 A, Bhowmick
- US 20080269157 A, Srivastava
- US 20080222741 A, Chinnaiyan
- US 20080181850 A, Thaxton
- US 20080014590 A, Dahary
- US 20060269971 A, Diamandis
- US 20060234259 A, Rubin
- US 20060115821 A, Einstein
- US 20060110759 A, Paris
- US 20040053247 A, Condon-Cardo
- US 20090127454 A, Ritchie
- US 7476506 B, Schleyer
- US 7473531 B, Domon
- US 7358231 B, McCaffey
- US 20050095611 A, Chan
- US 20060258841 A, Mitchl
- US 7473530 B, Hutteman
- US 7332590 B, Nacht
- US 7314721 B, Gure
- US 6576420 B, Carson
- US 20090062144 A, Guo
- US 20080176236 A, Tsao
- US 20080050378 A, Nakamura
- US 20060252057 A, Raponi
- US 20060223127 A, Yip
- US 20060046257 A, Pollock
- US 20030224509 A, Moon
- US 20090098543 A, Budiman
- US 7442507 B, Polsky
- US 20080286811 A, Moses
- US 20080268473 A, Moses
- US 20030232356 A, Dooley
- US 20080274908 A, Chang
- US 20080118462 A, Alani
- US 20070154889 A, Wang
- US 20080064047 A, Zetter
- US 7449303 B, Coignet
- US 7308364 B, Shaughnessy
- US 7049072 B, Seshi
- US 20080293578 A, Shaughnessy
- US 20080234139 A
- US 20080234138 A
- US 7479371 B, Ando
- US 7479370 B, Coignet
- US 7416851 B, Davi
- US 7316906 B, Chiorazzi
- US 6130052 A, Van Baren
- US 5643729 A, Taniguchi
- US 20090131353 A, Insel
- US 7332280 B, Levy
- US 5858655 A, Arnold
- US 6936417 B, Orntoft
- US 6008003 A, Haak-Frendscho
- US 6998232 B, Feinstein
- US 20080311604 A, Elting
- US 20090029372 A, Wewer

**Non-patent literature cited in the description**

- **EIJK et al.** *Meth. mol. Biology,* 2011, vol. 688, 97-126
- **GUO et al.** *Clin. Chem.,* 2010, vol. 56, 1451-1459
- **MANIATIS et al.** Molecular Cloning: A Laboratory Manual. Cold Spring Harbor, 1982, 280-281
- **SAMBROOK et al.** DNA microarray: A Molecular Cloning Manual. Cold Spring Harbor, 2003
- **MANIATIS et al.** Molecular Cloning: A Laboratory Manual. Cold Spring Harbor, 1982
- **CARUTHERS.** *Science,* 1985, vol. 230, 281-285
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **JOOS et al.** *Analytical Biochemistry,* 1997, vol. 247, 96-101

- **OROSKAR et al.** *Clin. Chem.,* 1996, vol. 42, 1547-1555
- **KHANDJIAN.** *Mol. Bio. Rep.,* 1986, vol. 11, 107-115
- **TAYLOR et al.** *J. Phys. D. Appl. Phys.,* 1991, vol. 24, 1443
- **SMITH et al.** *Science,* 1992, vol. 253, 1122
- **HARRIS T. D. et al.** *Science,* 2008, vol. 320, 106-109
- **BRASLAVSKY et al.** *PNAS (USA),* 2003, vol. 100, 3960-3964
- **MARGULIES, M et al.** *Nature,* 2005, vol. 437, 376-380
- **SONI G V ; MELLER A.** *Clin Chem,* 2007, vol. 53, 1996-2001
- **MOUDRIANAKIS E. N. ; BEER M.** *Proc Natl Acad Sci USA.,* March 1965, vol. 53, 564-71
- **DIEFFENBACH ; DVEKSLER.** PCR Primer, a Laboratory Manual. Cold Spring Harbor Press, 1995
- **BARANY F.** *PNAS,* 1991, vol. 88, 189-193
- **BARANY F.** *PCR Methods and Applications,* 1991, vol. 1, 5-16
- **NARANG et al.** *Methods Enzymol.,* 1979, vol. 68, 90
- **BROWN et al.** *Methods Enzymol.,* 1979, vol. 68, 109
- **CUNNINGHAM et al.** Williams Obstetrics. McGraw-Hill, 2002, 942
- **FAN et al.** *PNAS,* 2008, vol. 105 (42), 16266-16271
- **HESKETH.** The Oncogene Facts Book. Academic Press Limited, 1995
- **HELLMAN et al.** *Br J Cancer.,* 2009, vol. 100 (8), 1303-1314
- **FILELLA et al.** *Acta Oncol.,* 1994, vol. 33 (7), 747-751
- **LIPKIN et al.** *Cancer Research,* 1988, vol. 48, 235-245
- **TCHAGANG et al.** *Mol Cancer Ther,* 2008, vol. 7, 27-37
- **HASINA et al.** *Cancer Research,* 2003, vol. 63, 555-559
- **RALHAN.** *Mol Cell Proteomics,* 2008, vol. 7 (6), 1162-1173
- **FACA et al.** *PLoS Med,* 2008, vol. 5 (6), e123
- **VAN BOCKSTAELE et al.** *Blood Rev.,* 2009, vol. 23 (1), 25-47